(19) Europäisches Patentamt
European Patent Office
Office européen des brevets

(11) Publication number: **0 074 306**
**B1**

(12) # EUROPEAN PATENT SPECIFICATION

(45) Date of publication of patent specification: **01.06.88**

(21) Application number: **82401571.3**

(22) Date of filing: **24.08.82**

(51) Int. Cl.$^4$: **C 07 D 473/18,** C 07 F 9/65, C 12 P 17/12, A 61 K 31/52 // C07C69/12, C07D319/06, C07D317/24

(54) **Anti-viral guanine derivatives.**

(30) Priority: **26.08.81 US 296604**

(43) Date of publication of application:
**16.03.83 Bulletin 83/11**

(45) Publication of the grant of the patent:
**01.06.88 Bulletin 88/22**

(84) Designated Contracting States:
**AT BE CH DE FR GB IT LI LU NL SE**

(56) References cited:
**EP-A-0 049 072**
**EP-A-0 066 208**
**US-A-4 146 715**

**TETRAHEDRON LETTERS, vol. 21, 1980, Pergamon Press Ltd. OXFORD (GB). K.K. OGILVIE et al.: "Ring open analogues of deoxynucleotides", pages 327-330**

**CHEMICAL ABSTRACTS, vol. 97, no. 15, 11th October 1982, page 27, abstract no. 120161c, COLUMBUS OHIO (US).**

**The file contains technical information submitted after the application was filed and not included in this specification**

(73) Proprietor: **MERCK & CO. INC.**
**126, East Lincoln Avenue P.O. Box 2000**
**Rahway New Jersey 07065-0900 (US)**

(72) Inventor: **Ashton, Wallace T.**
**122 Sweet Briar Drive**
**Clark New Jersey 07066 (US)**
Inventor: **Karkas, John D.**
**404 W. 116th Street**
**New York New York 10027 (US)**
Inventor: **Field, Arthur K.**
**376 Meadowbrook Road**
**North Wales Pennsylvania 19454 (US)**
Inventor: **Tolman, Richard L.**
**29 Upper Warren Way**
**Warren New Jersey 07060 (US)**

(74) Representative: **Martin, Jean-Jacques et al**
**Cabinet REGIMBEAU 26, Avenue Kléber**
**F-75116 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 074 306 B1**

**Description**

Background of the Invention

The use of purine derivatives as anti-viral compounds is known. For example, U.S. Patent 4,027,025 discloses 8-azapurine derivatives such as 9-(2-hydroxyethoxymethyl)-8-azaguanine and 9-(2-benzoyloxyethoxymethyl)-8-azaguanine as anti-viral compounds.

U.S. Patent 4,146,715 discloses 2-amido-9-(2-acyloxyethoxymethyl)hypoxanthines.

U.S. Patent 4,199,574 discloses that 9-(2-hydroxyethoxymethyl) and related derivatives of certain 6-, and 2,6-substituted purines have anti-viral activity.

European patent application publication 0 049 072 discloses that 9-[[2-hydroxy-1-(hydroxymethyl)-ethoxy]methyl]guanine has anti-viral activity.

Objects of the Invention

It is an object of the present invention to provide novel, anti-viral compounds. Another object of the present invention is to provide novel compounds having enhanced anti-viral activity compared to known anti-viral compounds. Yet another object is to provide compounds having potent anti-viral activity against herpes viruses. Still another object is to provide compounds having antimycoplasmal activity. A further object of the present invention is to provide pharmaceutical formulations for the effective administration of the novel compounds of the invention. Still another object is to provide methods for the preparation of the novel compounds of the present invention. These and other objects of the present invention will become apparent from the following description.

Summary of the Invention

9-(1,3-dihydroxy-2-propoxymethyl)guanine and 9-(2,3-dihydroxy-1-propoxymethyl)guanine have been found to have potent anti-viral activities. These compounds, their acyl derivatives, their phosphate derivatives and their pharmaceutically acceptable salts, pharmaceutical formulations containing these compounds, the treatment of viral infections with these compounds, methods of preparing these compounds, and novel intermediates useful in their preparation are all disclosed. In addition, the acyl derivatives have antimycoplasmal activity.

The compounds of the present invention may be prepared by reaction of the appropriate acetoxymethyl ether with diacetylguanine, followed by deprotection. The acetoxymethyl ethers may be obtained by reaction of glycerol formal with acetic anhydride in the presence of a catalyst.

Brief Description of the Drawings

Figure 1 shows phosphorylation reaction velocity versus concentration curves; and

Figure 2 is a bar graph showing results of treating HSV—1 infected mice with acycloguanosine or the compound of formula II.

Detailed Description

The present invention relates to a compound of the formula:

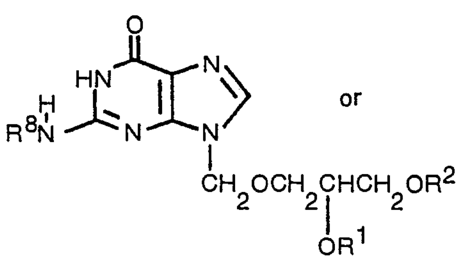

or

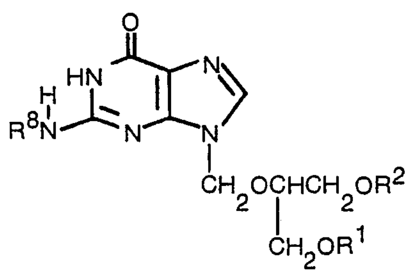

I II

and the pharmaceutically acceptable salts thereof, wherein $R^1$ and $R^2$ are independently H,

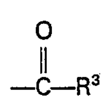

or

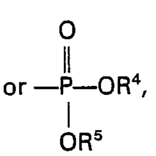

or $OR^1$ and $OR^2$ together are

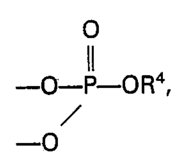

wherein $R^3$ is H, alkyl of 1—20 carbon atoms, preferably 1—10 carbon atoms, which may be straight chain or branched, saturated or mono- or polyunsaturated, phenyl, optionally substituted by halogen or $C_{1-4}$ alkyl, pyridyl, piperidyl, furyl, imidazolyl, tetrahydrofuryl or thienyl, $C_{1-4}$ alkyl substituted by phenyl, alkoxyalkyl, wherein both the alkoxy and alkyl groups contain 1—4 carbon atoms, or $C_{1-4}$ alkyl substituted by phenoxy; $R^4$ and $R^5$ are independently H, a pharmaceutically acceptable cation, alkyl of 1 to 8 carbon atoms which may be straight chain or branched, phenyl optionally substituted by halogen or $C_{1-4}$ alkyl, phosphate or pyrophosphate, and $R^8$ is H or

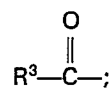

with the proviso that $R^1$, $R^2$ and $R^8$ do not simultaneously represent H in the formula II and that $R^8$ is not acyl when $R^1$ and $R^2$ are H.

Preferably the alkyl group is from 1—10 carbon atoms, and the pharmaceutically acceptable cation is sodium, potassium, ammonium, alkyl ($C_{1-4}$) substituted ammonium, magnesium/2, calcium/2, or aluminum/3.

The compounds of formulas I and II may be prepared starting from glycerol formal, a mixture of 1,3-dioxan-5-ol of the formula

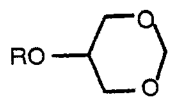

III

wherein R is H and 1,3-dioxolane-4-methanol of the formula

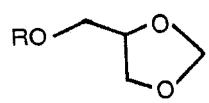

IV

wherein R is H, of which the compound of formula III is normally the predominant species. The ratio of compounds of formulas III and IV in glycerol formal has been determined to be 57:43 by H. Tibert, Fresenius' Z. Anal. Chem., *265,* 328 (1973). This ratio may vary, however, for different preparations of glycerol formal. It is to be understood that mixtures containing various ratios of the compounds of formulas III and IV may be employed according to the present invention.

The glycerol formal (mixture of compounds of formulas III and IV) is preferably acylated, e.g. by reaction with an acylating agent such as acetic anhydride in the presence of pyridine, without separation of the individual compounds of formulas III and IV, to give the corresponding acyloxy derivatives wherein R is acyl. This mixture is separated, e.g. by high performance liquid chromatography (HPLC).

Treatment of the compound of formula III wherein R is Ac (acetyl) with acetic anhydride in the presence of a catalyst, e.g. $ZnCl_2$, gives acetoxymethyl 2,3-diacetoxy-1-propyl ether of formula

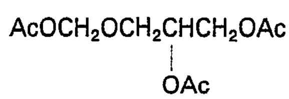

V

This reaction is exothermic and takes place at about ambient temperature preferably in an inert atmosphere, e.g. $N_2$.

The compound of formula V is then purified and reacted neat or in an inert solvent such as triglyme with diacetylguanine, prepared as described by Ishido et al., Bull. Chem. Soc. Japan, *37,* 1389 (1964), of the formula

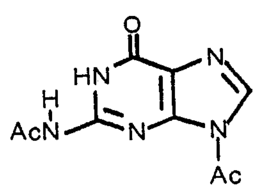

(VII)

at elevated temperature under vacuum in the presence of an acidic catalyst, e.g. ethanesulfonic acid, to form 2-acetamido-9-(2,3-diacetoxy-1-propoxymethyl)hypoxanthine VIII of the formula

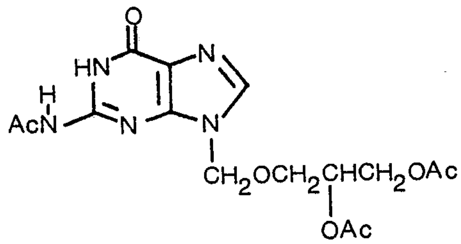

VIII

as a viscous oil. The oil is taken up in a suitable solvent e.g. ethyl acetate, and allowed to crystallize.

The compound of formula VIII is then deacetylated, e.g. by heating with aqueous methylamine under reflux preferably in an inert atmosphere, e.g. $N_2$, and then cooled to yield a solution containing the compound of formula I which is optionally treated with charcoal and filtered. Concentration of the filtrate gives a solid which is recrystallized from $H_2O$ to give a crystalline product.

The compound of formula II may be obtained in similar fashion by reacting the compound of formula IV wherein R is Ac with acetic anhydride in the presence of a catalyst, e.g. $ZnCl_2$, to give acetoxymethyl 1,3-diacetoxy-2-propyl ether of the formula

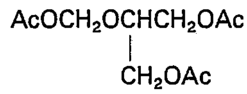

VI

This reaction takes place under similar conditions as used to form compound V from compound III wherein R is Ac.

The compound of formula VI is then purified and reacted with diacetylguanine of formula VII, under conditions similar to those used to react the compound of formula V with diacetylguanine, to form 2-acetamido-9-(1,3-diacetoxy-2-propoxymethyl)hypoxanthine of formula IX as a viscous oil which is crystallized by a procedure similar to that employed for the compound of formula VIII.

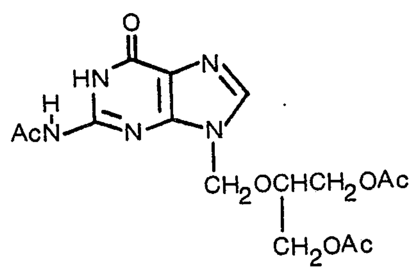

IX

The compound of formula IX is then converted to the crystalline product of formula II by treatment similar to that employed to convert the compound of formula VIII to formula I.

It is also possible if desired, to separate glycerol formal into its constituent compounds of formulas III and IV wherein R in each case is H and then to treat each separated compound as described above.

It is equally possible to form a mixture of compounds of formulas V and VI from glycerol formal by treating the latter directly with acetic anhydride in the presence of a catalyst, e.g., $ZnCl_2$. The resulting mixture may be chromatographed by HPLC. Fractions showing only the compounds of formula V by analytical HPLC are concentrated under high vacuum and then reacted with diacetylguanine of formula VII as described above to give the compound of formula VIII which in turn is converted to the compound of formula I as described above.

Alternatively, the acetyl intermediates of formulas V and VI, each separately or as a mixture, can be converted to the more reactive halogen derivatives by treatment with hydrogen halide in a nonpolar solvent (hydrogen chloride in dichloromethane is preferred) wherein the terminal $AcOCH_2O$— functionality is transformed to $XCH_2O$— where X is a halogen (chlorine, bromine, or iodine). These halogen compounds can be used in alkylation reactions with protected guanines [per(trimethylsilyl)guanine or diacetylguanine are two preferred derivatives] in nonpolar or dipolar solvents such as benzene, toluene, acetonitrile or dimethylformamide with or without an acid-acceptor substance such as triethylamine or powdered calcium carbonate as has been described in the literature, for example, in U.S. Patent 4,199,574.

Chromatographic fractions containing primarily the compound of formula VI are rechromatographed by HPLC and those fractions of satisfactory purity are combined, concentrated under high vacuum and then reacted with diacetylguanine of formula VII as described above to give the compound of formula IX which in turn is converted to the compound of formula II as described above.

While the foregoing process description has referred specifically to compounds wherein the acyl group is acetyl, it is to be understood that the acyl group may equally be a straight or branched chain alkanoyl group of up to 20 carbon atoms, which may be saturated or mono- or polyunsaturated or phenyl, optionally

substituted by halogen or $C_{1-4}$ alkyl, pyridyl, piperidyl, furyl, imidazolyl, tetrahydrofuryl or thienyl, $C_{1-4}$ alkyl substituted by phenyl, alkoxyalkyl, wherein both the alkoxy and alkyl groups contain 1—4 carbon atoms, or $C_{1-4}$ alkyl substituted by phenoxy.

According to the present invention, the acyl derivatives are prepared by reacting a compound of formula I or II wherein $R^1$, $R^2$ and $R^8$ are each H with an acylating compound of the formula

$$R^3\overset{\overset{O}{\|}}{C}X$$

wherein X is a carboxyl activating group whereby a monoacylated derivative is formed when the reaction is allowed to proceed until 1 equivalent of the acylating compound is reacted with the compound of formula I or II, or whereby a diacylated derivative is formed when the reaction is allowed to proceed until 2 equivalents of the acylating compound are reacted with the compound of formula I or II or whereby a triacylated derivative is formed when 3 equivalents of the acylating agent are reacted with the compound of formula I or II.

The acyl derivatives are preferably prepared by reacting the compounds of formulas I or II with the appropriate acyl halide, acid anhydride, or other activated acyl species in the presence of an appropriate cosolvent such as, for example, pyridinedimethylformamide. 4-Dimethylaminopyridine is an effective catalyst. Other activated acyl species may be prepared by reaction of the acid with a suitable activating agent such as, for example, 1,1′-carbonyldiimidazole, N,N′-dicyclohexylcarbodiimide or by acylation of N-hydroxysuccinimide 1-hydroxy-benzotriazole.

Compared to acycloguanosine, 9-(2-hydroxyethoxymethyl)guanine, the compounds of the present invention are more soluble and are more readily phosphorylated by viral enzymes, and have substantially greater activity *in vivo* than acycloguanosine. The compounds of the present invention may be employed as anti-viral compounds in mammalian or avian species either individually or in combination in dosage levels effective to impart an anti-herpes virus activity. Typically such levels are from about 0.01 to about 200 mg/kg/day. The compounds of the present invention may be formulated according to accepted pharmaceutical practice for administration orally, topically or by injection. In particular, for topically administration, suitable dosage level are from about 0.1% to about 5% by weight, more particularly from about 0.25% to about 3% by weight. For orally or parenterally administration, suitable dosage level are from about 0.8 to about 100 mg/kg, more preferably from about 5 to about 50 mg/kg. Suitable oral dosage forms are tablets, capsules, elixirs or powders, while solutions or suspensions in, for example, phosphate buffered saline or water are suitable for injection. Examples of suitable topical formulations are gels, ointments, solutions or suspensions.

The acyl derivatives ($C_{1-20}$) of the compounds of the present invention have antimycoplasmal activity and are useful in treating or preventing this disease in swine and poultry.

The following examples illustrate the present invention. All temperatures are expressed in degrees Celsius.

Example 1

9-(2,3-Dihydroxy-1-propoxymethyl)guanine

A. Acetoxymethyl 2,3-Diacetoxy-1-propyl Ether

To a stirred mixture of 17.1 ml (20.8 g, 200 mmole) of glycerol formal containing a mixture of the compounds of formulas III and IV, there was added 60 ml of acetic anhydride, 6.7 ml of glacial acetic acid, and 2.0 g of anhydrous $ZnCl_2$. The mixture was stirred at ambient temperature under an $N_2$ atmosphère. The $ZnCl_2$ soon dissolved, and within a few minutes thère was a strong exothermic reaction with the color of the solution turning light amber. After one hour, by which time the exotermic reaction had subsided, thin layer chromatography (TLC) (1:1 and 2:1 hexane-ethyl acetate) showed an apparently complete and clean reaction. After 4.5 hours, the solution was concentrated under high vacuum. The residual oil was taken up in 700 ml of diethyl ether and washed with 2 × 100 ml of a saturated $NaHCO_3$ solution, and then with 100 ml of $H_2O$. The ether solution was dried over $MgSO_4$, decolorized with charcoal, and filtered. The filtrate was concentrated under high vacuum to give 45.8 g (92%) of colorless residual oil. Analytical HPLC (7:3 hexane-ethyl acetate) showed only the two product isomers of formula V and VI. The bulk of the two product isomers (44.5 g) was subjected to HPLC in four batches of from 11 to 11.5 g each. All runs were performed under identical conditions using silica gel (two Waters Prep-500 packs), 7:3 hexane-ethyl acetate (250 ml per minute flow rate) with recycling (three cycles) and refractive index detection. Fractions were cut in the same place for each run, and fractions from the various runs were combined as they were collected. No clear separation of peaks was observed on the refractive index trace.

Fractions identified by analytical HPLC as pure compound of formula V (shorter retention time) were combined and concentrated under high vacuum to give 17.0 g of a colorless residual oil whose NMR ($CDCl_3$) was consistent for acetoxymethyl 2,3-diacetoxy-1-propyl ether.

B. 2-Acetamido-9-(2,3-diacetoxy-1-propoxymethyl)hypoxanthine

A mixture of 2.61 g (11.1 mmole) of diacetylguanine (VII), 5.50 g (22.2 mmole) of acetoxymethyl 2,3-

diacetoxy-1-propyl ether from Step A above and 55 mg of ethanesulfonic acid was heated in a flask fitted with a distillation adapter under low vacuum in an oil bath at 155—160°. The mixture gradually thinned enough to permit magnetic stirring, and some distillate was collected. The mixture became homogenous after about 45 minutes and was cooled after 75 minutes. The viscous oil was taken up in about 100 ml of ethyl acetate and induced to crystallize with a yield of 1.23 g (29%) of nearly white crystals, mp 162.5—165°. Thin layer chromatography (TLC) (9:1 $CHCl_3$—$CH_3OH$) showed a single spot.

C. 9-(2,3-Dihydroxy-1-propoxymethyl)guanine

A solution of 1.14 g (3.0 mmole) of 2-acetamido-9-(2,3-diacetoxy-1-propoxymethyl)hypoxanthine (VIII) from Step B above was heated at reflux in 40% aqueous methylamine with stirring under $N_2$ for 1 hour and then cooled. TLC (80:20:2 $CHCl_3$—$CH_3OH$—$H_2O$) showed complete conversion to the title compound (I). The light orange solution was treated with some charcoal and filtered through Super-Cel®. Concentration of the filtrate gave a solid which was recrystallized from $H_2O$ (adjusted to about pH 6 with a few drops of $CH_3COOH$) to yield 687 mg of cream-colored crystals, mp 246—247° dec.

Example 2

9-(1,3-Dihydroxy-2-propoxymethyl)guanine

A. Acetoxymethyl 1,3-Diacetoxy-2-propyl Ether

After drying under high vacuum, fractions from step A of Example 1 which were high in content of formula VI (longer retention time) were combined to give 9.71 g of residual oil which was subjected to HPLC under the same conditions as described above. Fractions containing the compound of formula VI in satisfactory purity as determined by analytical HPLC were combined and concentrated under high vacuum to give 5.01 g of an almost colorless residual oil. Analytical HPLC indicated a ratio of compound of formula VI to the compound of formula V of approximately 15:1 based on peak heights. The NMR ($CDCl_3$) was consistent with this isomer ratio and was in accord with the identification of this compound as acetoxymethyl 1,3-diacetoxy-2-propyl ether.

B. 2-Acetamido-9-(1,3-diacetoxy-2-propoxymethyl)hypoxanthine

A mixture of 3.76 g (16 mmole) of diacetylguanine (VII), 4.96 g (20 mmole) of acetoxymethyl 1,3-diacetoxy-2-propyl ether (VI), 40 mg of ethanesulfonic acid, and 15 ml of triglyme in a flask fitted with a distillation adapter was heated under low vacuum in an oil bath at 155—160°. The mixture gradually thinned enough to stir, and a clear distillate was slowly collected. The reaction mixture became a clear solution after about 75 minutes. After 3 hours the solution was cooled, and the product was induced to crystallize. After standing, the thick mixture was diluted with a small volume of 1,2-dimethoxyethane. The solid was collected on a filter and washed with small volumes of 1,2-dimethoxyethane followed by ethyl acetate to give 3.27 g of cream-colored crystals consisting of a mixture of 9- and 7-alkylated isomers as determined by TLC (9:1 $CHCl_3$—MeOH). (The 9-isomer runs more slowly than the 7-isomer in this system). The mother liquor provided an additional 0.65 g of material. The combined crops (3.92 g) were chromatographed twice on a silica gel column (elution with 97:3 and then 96:4 $CH_2Cl_2$—MeOH). Fractions containing nearly pure 9-alkylated isomer were combined and concentrated. Crystallization of the residues from a minimum amount of 1,2-dimethoxyethane yielded a first crop of 665 mg (white crystals, mp 171.5—172.5°) and a second crop of 189 mg (mp 173—173.5°). Both crops consisted of the pure 9-alkylated product as determined by TLC in comparison with an earlier batch fully characterized by NMR and elemental analysis.

C. 9-(1,3-Dihydroxy-2-propoxymethyl)guanine

A solution of 838 mg (2.2 mmole) of 2-acetamido-9-(1,3-diacetoxy-2-propoxymethyl)hypoxanthine in 8.5 ml of 40% methylamine (aqueous) was stirred at gentle reflux under $N_2$ for 1 hour. The solution was then cooled and concentrated to dryness. The residual white solid was recrystallized from a minimum volume of $H_2O$ containing 2 drops of acetic acid. After standing in the refrigerator, the product was collected on a filter and washed with a small amount of $H_2O$, then acetone. The material was dried under high vacuum at 75° for 3 hours to give 529 mg (90% based on hydration with 0.75 $H_2O$) of white crystals, mp 249—250° dec. The material was homogeneous by TLC (80:20:2 $CHCl_3$—MeOH—$H_2O$), and the structure was confirmed by NMR.

Example 3

Acetoxymethyl 1,3-Diacetoxy-2-propyl Ether

A. 1,3-Dioxan-5-yl Acetate and 1,3-Dioxolane-4-methyl Acetate

A mixture of 10.0 g (96 mmole) of glycerol formal, 8.35 g (105 mmole) of pyridine, and 20 ml of acetic anhydride was stirred at ambient temperature under protection from moisture. After a period ranging from a few hours to 5 days, the solution was fractionally distilled under vacuum. The early fractions consisted primarily of pyridine, acetic acid, and acetic anhydride. The bulk of the product distilled at 56—57° (1.1 mm). The product fractions (10.8 g) were separated into the 5- and 6-membered ring isomers by preparative HPLC on silica gel in 3:1 hexane-ethyl acetate with recycling. Fractions were identified and checked for purity by analytical HPLC. In total 3.19 g (23%) of 1,3-dioxolane-4-methyl acetate (shorter retention time)

and 5.23 g (37%) of 1,3-dioxan-5-yl acetate (longer retention time) were obtained. The structures were confirmed by NMR.

B. Acetoxymethyl 1,3-Diacetoxy-2-propyl Ether

A solution of 6.0 g (41 mmole) of 1,3-dioxolane-4-methyl acetate and 0.4 g of zinc chloride in a mixture of 12 ml of acetic anhydride and 1.4 ml of glacial acetic acid was stirred at ambient temperature under $N_2$. An exotherm occurred, and after 1 hour TLC (2:1 hexane-ethyl acetate) indicated complete reaction. The solution was concentrated under high vacuum. The resulting liquid was dissolved in ether and washed thoroughly with saturated $NaHCO_3$ solution, then with $H_2O$. The ether layer was dried over $MgSO_4$, filtered, and concentrated to give 8.68 g of residual oil consisting of a mixture of the compound of formula VI (major) and the compound of formula V (minor). This material was combined with 3.50 g from a similar batch. The total of 12.18 g of crude product was purified by preparative HPLC on silica gel in 7:3 hexane-ethyl acetate with recycling. The fractions were checked for purity by analytical HPLC. Fractions of satisfactory purity were combined and concentrated to give 5.84 g of the compound of structure VI ($\geq$ 90% pure). The structure and purity were confirmed by NMR.

### Example 4
### Bromomethyl 1,3-Diacetoxy-2-propyl Ether

Acetoxymethyl 1,3-diacetoxy-2-propyl ether (250 mg, 1 mmole) was dissolved in dichloromethane presaturated with hydrogen bromide gas at 0°. The mixture was protected from moisture and stirred at 0° for 2 hours, then allowed to warm to ambient temperature with loss of excess hydrogen bromide. After three hours the solvents were removed under aspirator vacuum. The evaporation residue was treated successively with two 10 ml aliquots of dichloromethane, which were evaporated under aspirator vacuum. Finally the residual oil was dried under high vacuum until the sharp odor of hydrogen bromide was no longer evident. The resulting material may be used immediately in alkylation reactions. Proton magnetic resonance spectra of $CDCl_3$ solutions showed an appropriate downfield shift reflecting the change from $AcOCH_2O$ to $BrCH_2O$.

### Example 5
### Chloromethyl 1,3-Diacetoxy-2-propyl Ether

A solution of 4.34 g (17.5 mmole) of acetoxymethyl 1,3-diacetoxy-2-propyl ether in 45 ml of methylene chloride was stirred at room temperature as a gentle stream of HCl was passed through it. After 2 hours the HCl stream was removed. The flask was stoppered and allowed to stir overnight at room temperature. Then the flask was placed in a water bath at 25—30°, and the solution was purged with a stream of $N_2$ to remove most of the excess HCl. The remaining solution was concentrated by rotary evaporation. In order to remove traces of HCl, the residual oil was taken up in toluene and concentrated under high vacuum at room temperature. This process was repeated three more times. After vacuum drying at room temperature, the yield of colorless residual oil was 3.83 g (97%). The NMR spectrum indicated complete conversion to product.

### Example 6
### 2-Acetamido-9-(1,3-diacetoxy-2-propoxymethyl)hypoxanthine

A mixture of 2.57 g (18 mmole) of guanine, 1.8 g of ammonium sulfate, and 126 ml of hexamethyldisilazane was stirred at reflux under $N_2$. The solid gradually dissolved. After 2 days the solution was cooled and concentrated under high vacuum. The viscous, residual oil was dissolved in about 28 ml of dry toluene and maintained under $N_2$ as a solution of 5 g (22.3 mmole) of chloromethyl 1,3-diacetoxy-2-propyl ether in 12 ml of dry toluene was added. The resulting solution was heated at reflux under $N_2$ for 1.5 hours. It was then cooled, concentrated, and dried under high vacuum. The viscous, orange residual oil was treated with 30 ml of water and 30 ml of saturated sodium bicarbonate solution. The mixture was swirled with warming on a steam bath for 5 minutes, during which time the residue solidified. After cooling, the solid was collected on a filter and washed with a small volume of water. Although this cream-colored solid (4.6 g) gave a single spot on TLC (80:20:2 $CHCl_2$—MeOH—$H_2O$), NMR showed that it contained 10—15% of the 7-alkylated isomer in addition to the desired 9-isomer. The material was combined with 0.6 g of similar material from other runs and was suspended in 184 ml of acetic anhydride. The mixture was heated at 97° for 18 hours by which time nearly all of the solid had dissolved and TLC showed complete acetylation. The reaction was cooled and concentrated under vacuum. Treatment of the residue with 200 ml of methylene chloride gave a solid, which was isolated by filtration and washed once with methylene chloride. Recrystallization from methylene chloride gave 0.55 g of pure 9-isomer. The filtrates were passed through a column containing 40 g of silica gel. Elution with 97:3 $CH_2Cl_2$—MeOH gave 4.5 g of partially purified product. Upon recrystallization from methylene chloride (about 45 ml), 3.0 g of pure 9-isomer was obtained.

### Example 7

9-(1,3-Diacetoxy-2-propoxymethyl)quanine

A mixture of 50.0 g (0.33 mole) of guanine, 33 g of ammonium sulfate, and 2.2 l of hexamethyl-

disilazane was stirred at reflux under $N_2$ for 3 days, during which time all of the solid dissolved. The solvent was then removed by distillation under reduced pressure. To the very viscous, orange residual oil was added under $N_2$ 84 g (0.34 mole) of acetoxymethyl 1,3-diacetoxy-2-propyl ether, which formed a second liquid phase at the bottom of the flask. The flask was fitted with a distillation adapter, and the mixture was heated under low vacuum in an oil bath at approximately 135°. After an induction period lasting several minutes, boiling began and soon became quite vigorous. Distillation of trimethylsilyl acetate (along with any residual hexamethyldisilazane) proceeded rapidly at first but slowed after 30 minutes. After 2 hours the mixture was added to 1.3 l of 90% EtOH. The mixture was heated to boiling and maintained there until the separateed gummy material was transformed to a tractable solid. The solid (8.2 g, consisting almost exclusively of quanine) was removed by filtration while hot. The filtrate was allowed to stand overnight, resulting in separation of an orange-brown gum. The supernatant was decanted away from the gum, filtered, and then concentrated to small volume. The solid which separated on concentration was collected on a filter and washed with $H_2O$, then with some EtOH, to give 15.4 g of cream-colored crystals. by NMR, this material was solely the 9-alkylated isomer, although TLC indicated partial side chain deacetylation. The material was suitable for deprotection without further purification.

Further processing of the mother liquor, and of the gum which had been removed by decantation, gave additional crops consisting of varying ratios of 9- and 7-alkylated isomers (partially deacetylated). These less pure crops were preferably converted to fully acetylated derivatives (0,0',$N^2$-triacetyl) prior to chromatographic purification (silica gel, elution with $CH_2Cl_2$-MeOH). Typical acetylation conditions consisted of stirring a mixture of 10 g of the crude guanine derivative and 400 ml of acetic anhydride at 95—100° overnight, followed by concentration and chromatography.

## Example 8

9-(1,3-Diacetoxy-2-propoxymethyl)quanine

A mixture of 205 mg (0.75 mmole) of 9-(1,3-dihydroxy-2-propoxymethyl)guanine monohydrate, 1.5 ml of acetic anhydride, 6 ml of dry dimethylformamide, and 1.5 ml of dry pyridine was stirred at room temperature under a drying tube for 4 days. Then the mixture was diluted with 15 ml of $Et_2O$. The solid was collected on a filter and washed with $Et_2O$. After recrystallization from 2-methoxyethanol, yield of colorless crystals = 149 mg (59%), m.p. 239—240°. The material was homogenous by TLC (9:1 $CHCl_3$-MeOH). and NMR confirmed the assigned structure.

Anal. ($C_{13}H_{17}N_5O_6$).
Calcd.: C, 46.01; H, 5.05; N, 20.64.
Found: C, 45.71; H, 5.04; N, 20.36.

## Example 9

9-(1,3-Dipropionyloxy-2-propoxymethyl)guanine

A mixture of 205 mg (0.75 mmole) of 9-(1,3-dihydroxy-2-propoxymethyl)guanine monohydrate, 1.5 ml of propionic anhydride, 6 ml of dry dimethylformamide, and 1.5 ml of dry pyridine was stirred at room temperature under a drying tube. After 4 days the mixture was diluted with 25 ml of ether. The solid was collected on a filter and washed with ether. Recrystallization from isopropanbol gave 136 mg (50%) of white crystals, m.p. 196—197.5°. The material ran as a single spot on TLC (9:1 $CHCl_3$-MeOH), and the structure was confirmed by NMR.

Anal. ($C_{15}H_{21}N_5O_6$).
Calcd.: C, 49.04; H, 5.76; N, 19.07.
Found: C, 48.96; H, 5.83; N, 19.26.

## Example 10

9-(1-Hydroxy-3-octanoyloxy-2-propoxymethyl)guanine

A suspension of 410 mg (1.5 mmole) of 9-(1,3-dihydroxy-2-propoxymethyl)guanine monohydrate in 6 ml of dry dimethylformamide and 1.5 ml of dry pyridine was stirred under a drying tube with cooling in an ice bath as a solution of 489 mg (3.0 mmole) of octanoyl chloride in 1.5 ml of dimethylformamide was added dropwise by syringe over approximately 5 minutes. The mixture was allowed to warm gradually to room temperature, and after 24 hours it was concentrated under high vacuum. The residual oil was purified by preparative TLC on nine 1000-μ silica gel plates (developed in 5:1 $CHCl_3$-MeOH). The product bands were isolated, combined, and extracted with dimethylformamide. Concentration of the extracts under high vacuum gave a gummy residue. Crystallization from isopropanol gave a material which again turned gummy on the filter. However, thorough trituration with ether yielded 105 mg (18%) of very pale yellow crystals, m.p. 201.5—203.5°.

## Example 11

9-(1,3-Dioctanoyloxy-2-propoxymethyl)guanine

A suspension of 137 mg (0.5 mmole) of 9-(1,3-dihydroxy-2-propoxymethyl)guanine monohydrate in 2.8 ml of dry dimethylformamide and 0.7 ml of dry pyridine was stirred at room temperature as 244 mg (1.5 mmole) of octanoyl chloride was added. The addition was accompanied by a mild exotherm, and a clear solution was obtained. An additional 82 mg (0.5 mmole) of octanoyl chloride was added after 3.5 hours.

Finally, after 21 hours, the solution was concentrated under high vacuum. The residue was partitioned between 5 ml of methylene chloride and 5 ml of water. The methylene chloride phase was dried over magnesium sulfate, filtered, and concentrated to give a pale yellow residual oil, which solidified on standing. This material was purified by preparative TLC on five 2000-μ silica gel plates (developed in 12:1 $CHCl_3$-MeOH). The product bands were isolated and extracted with methanol. The residue obtained on concentration of the extracts was taken up in ether and filtered. Evaporation of the filtrate followed by drying under high vacuum yielded 162 mg (64%) of pale yellow-orange, glossy residue. Purity and structure were confirmed by TLC (12:1 $CHCl_3$-MeOH), NMR, and mass spectrum.

This compound gave a 75% inhibition of mycoplasmal growth in chickens when administered systemically at a dosage level of 875 μg/bird.

Example 12

Sodium 9-(1,3-Dihydroxy-2-propoxymethyl)guanine cyclic monophosphate

A suspension of 5.91 g (23.2 mmoles) of anhydrous 9-(1,3-dihydroxy-2-propoxymethyl)-guanine in a solution of 3.6 g (2.2 ml; 23.6 mmoles) of phosphorus oxychloride in 60 ml of anhydrous triethyl phosphate was stirred at room temperature for five hours. The largely clarified mixture was filtered, and the filtrate was poured into 600 ml of stirred hexane. After about five minutes the supernatant hexane was decanted from the precipitated product, and the residue was heated with a second 600 ml portion of hexane. After the supernatant hexane was decanted and the residue was dried *in vacuo*, 15.9 g of a solid product was obtained. The solid was largely dissolved in 800 ml of deionized water and the cloudly mixture was titrated to pH 7 with 5*N* potassium hydroxide and then 1N potassium hydroxide. The neutralized mixture was filtered and the filtrate was lyophilized yielding 9.25 g of product.

A specimen of the lyophilization residue was analyzed by high performance liquid chromatography using a Whatman Partisil® PXS 10/25 SAX ion exchange column with 0.05M pH 6.6 phosphate buffer elution and ultraviolet absorption detection at 252 nm. The product exhibited three peaks with retention times of about 4 minutes, 7 minutes and 9 minutes. After authentic specimens of sodium and potassium 9-(1,3-dihydro-2-propoxymethyl)guanine *cyclic* and acylic monophosphate were isolated as disclosed in this and other examples in this patent application and subjected to high performance liquid chromatography in the above system, the *cyclic* monophosphate was associated with a retention time of about 4 minutes and the acyclic phosphate with a retention time of about 7 minutes.

The lyophilized mixture of potassium salts was dissolved in 1 liter of deionized water and filtered through a fluted filter paper. The filtrate was slowly passed through a 4—5 cm diameter counn of 460 ml (644-milliequivalents) of 200—400 mesh Bio Rad® AGI—X8 anion exchange resin on the bicarbonate cycle. Next, a gradient of 0.05M—0.5M potassium bicarbonate from a gradient elution chamber containing 2 liters of 0.05M and 0.5M potassium bicarbonate was pumped through the column and fractions of about 20 ml were collected at 8-minute intervals. At fraction 191, the eluent was changed to 0.5M potassium bicarbonate and samples of 20—25 ml were collected at 6.8-minute intervals. The elution pattern was monitored by ultraviolet absorption at 252 nm and certain component fractions of the various elution peaks were further characterized by high performance liquid chromatography in the Whatman Partisil® PXS 10/25 SAX ion exchange column using 0.05M pH 6.6 phosphate elution. On the basis of these data certain fractions were combined and worked up as follows:

Fractrions 450—540 (1680 ml) characterized by a single peak with a retention time of about 5 minutes in the above high performance liquid chromatography system, were combined and treated with 600 ml (1020 milliequivalents) of 200—400 mesh Bio Rad®AG50W-X8 cation exchange resin on the acid cycle. The stirred mixture was kept under a modest vacuum to remove carbon dioxide before it was filtered. The mixture was filtered and the resin was washed with small portions of deionized water. The combined filtrates were concentrated to a 150 ml volume *in vacuo* at about 35° and the precipitated product, in the form of the free acid, was isolated by filtration and dried *in vacuo* to yield 445 mg of 9-(1,3-dihydroxy-2-propoxymethyl)guanine cyclic monophosphate. The product was crystalline according to microscopy in polarized light, showed an ultraviolet absorption maximum at 252 nm (10600, in 0.1M pH 7 phosphate), and gave a nuclear magnetic resonance spectrum fully in accord with the projected structure. Concentration of the mother liquors yielded an additional 46 mg of the free acid form of the product. Titration of the mother liquors to pH 7 followed by lyophilization yielded 196 mg of sodium 9-(1,3-dihydroxy-2-propoxymethyl)quanine *cyclic* monophosphate. The latter compound, may at times be contaminated with small amounts of water soluble inorganic salts and may be purified by exclusion chromatography or by ion exchange chromatography on Bio Rad AGI—X8 anion exchange resin on the formate cycle.

The pure sodium salt was also obtained by titration of the crystalline free acid as follows:

A suspension of 213 mg of crystalline 9-(1,3-dihydroxy-2-propoxymethyl)quanine cyclic monophosphate was titrated to pH 7 with 1*N* sodium hydroxide and the solution was lyophilized yielding 227 mg of sodium 9-(1,3-dihydroxy-2-propoxymethyl) quanine cyclic monophosphate. A dried sample of this product had a ultraviolet absorptoin maximum at 252 nm (11800 in 0.1M pH 7 phosphate).

The 200 MHz NMR spectrum of the cyclic product in $D_2O$ is characterized by signals from two equivalent methylenes that have shifted downfield on monophosphorylation. The spectrum is characterized by the following chemical shifts.

| | | | | |
|---|---|---|---|---|
| δ 3.94 | 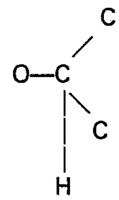 | m | 1H | |
| δ4.25 | P—O—CH eq | d,d,d | 2H | (JH,H gem 12.5 Hz)<br>(JP—OCH eq 19.5 Hz)<br>(JH,H *vic* eq 2.2 Hz) |
| δ4.39 | P—O—$CH_{ax}$ | d,d,d | 2H | (JH,H gem 12.5 Hz)<br>(JP—OCH ax 5.0 Hz)<br>(JH,H *vic* eq 2.2 Hz) |
| δ5.64 | N—$CH_2O$ | s | 2H | |
| δ7.99 | $C_8$—H | s | 1H | |

Additional confirmation of structure is obtained when the predicted pattern of shifts is realized for the P—O—$CH_2$ groups on irradiation of the

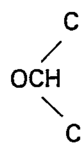 proton.

In a Varian AX—10 high performance liquid chromatography anion exchange column using a gradient of 10—1000 mM unbuffered $KH_2PO_4$, the cyclic product has a retention time of 4.3 minutes whereas the enzymically derived acyclic monophosphate has a retention time of 4.8 minutes. The synthetic cyclic monophosphate is clearly separated from the acyclic enzymically-derived monophosphate when a mixture of the two is subjected to HPLC in the above system.

As an alternative, the sodium or potassium salt of the cyclic monophosphate may be isolated from the Bio Rad AGI—X8 bicarbonate eluate fractions without isolation of the crystalline free acid. A combination of fractions amounting to about 800 ml of 0.5M $KHCO_3$ was treated with 325 ml (552 mmoles) of 200—400 mesh AG50W—X8 cation exchange resin on the acid cycle. The stirred mixture was kept under modest vacuum for fifteen minutes to remove carbon dioxide and was filtered. The filtrate was concentrated to about a 100 ml volume which was then titrated to pH 7 with 1N sodium hydroxide. Lyophilization of the resulting solution yielded 529 mg of the sodium salt of the cyclic phosphate that was contaminated with a small amount of water soluble inorganic salts.

To desalt the product, 200 mg of the sodium salt was dissolved in 1.5 ml of deionized water and put on a 1.5 cm diameter column of 6 ml of Bio Rad 200—400 mesh AG1—X8 anion exchange resin on the formate cycle. After about 35 ml of deionized water was passed through the column, elution was begun with 2*N* ammonium formate solution. Fractions of 3.5 ml volume were collected at 3 minute intervals and the ultraviolet absorption at 250 nm of each fraction was measured and plotted versus tube number. On the basis of the shape of the curve obtained in the above plot, fractions 13—28 were combined and put on a 2—3 cm diameter column of 120 ml (204 milliequivalents) of 200—400 mesh Bio Rad AG50W—X8 cation exchange resin. The column was eluted with water and 13.5 ml fractions were collected at 4.5-minute intervals. The elution pattern was monitored by ultraviolet absorption at 252 nm and on the basis of the plot, fractions 22—40 were combined and concentrated to dryness. The residue was taken up in 10 ml of deionized water and titrated to pH 7 with 0.1*N* NaOH. Lyophilization of the neutralized solution yielded 106 mg of sodium 9-(1,3-dihydroxy-2-propoxymethyl)guanine cyclic monophosphate.

## Example 13

### Disodium 9-(1,3-dihydroxy-2-propoxymethyl)guanine acyclic monophosphate

Preparation 1

In the phosphorylation of 9-(1,3-dihydroxy-2-propoxymethyl)guanine with phosphorus oxychloride to yield the corresponding cyclic monophosphate, the crude condensation product was purified by ion exchange chromatography on Bio Rad AG1—X8 ($CO_3^=$). In the course of elution with 0.5 M potassium bicarbonate as described in the example for preparation of the cyclic monophosphate, a discrete peak consisting of fractions 245—248 was separated and found to contain the corresponding acyclic compound dipotassium 9-(1,3-dihydroxy-2-propoxymethyl)guanine acyclic monophosphate. Using a Partisil™ PXS

10/25 SAX high performance liquid chromatography column and elution with 0.05M pH 6.6 phosphate buffer, this peak contained material with retention times of about 5 and 8 minutes. The authentic acyclic monophosphate is associated with a retention time of 7—8 minutes in the same system. Analysis of this combination of fractions on a Varian AX—10 high performance liquid chromatography anion exchange column using gradient elution with 10—400 mM unbuffered $KH_2PO_4$ showed that about 40% of the material was dipotassium 9-(1,3-dihydroxy-2-propoxymethyl)guanine acyclic monophosphate.

Preparation 2

A solution of 44.5 mg of sodium 9-(1,3-dihydroxy-2-propoxymethyl)guanine cyclic monophosphate in 4 ml of 5*N* sodium hydroxide was heated at 55—60°C under a nitrogen blanket for eight hours. The reaction mixture was diluted to a 12 ml volume with deionized water and passed slowly through a 30 ml (2 cm diameter X 12 cm length) column of Bio Rad AG50W—X8 cation exchange resin on the sulfonic acid cycle. The column was eluted with deionized water and 5 ml fractions were collected at 4-minute intervals. After fraction 60 was collected, 12 ml fractions were collected every 4 minutes. The various fractions were evaluated by ultraviolet absorption at 252 nm and also by high performance liquid chromatography on a Partisil™ PXS 10/25 SAX anion exchange column using 0.05M pH 6.6 phosphate buffer elution. Fractions 45—64, which consisted exclusively of material with a retention time of about 7.5 minutes were combined, titrated to pH 7 with 0.1*N* sodium hydroxide and then lyophilized to yield 30 mg of disodium 9-(1,3-dihydroxy-2-propoxymethyl)guanine acyclic monophosphate. A 200 MHz nuclear magnetic spectrum of the product in deuterium oxide is fully in accord with the acyclic monophosphate structure.

Anal. Calcd. for $C_9H_{12}N_5O_7PNa_2$ (379.19) N, 18.47; C, 28.51; H, 3.19; P, 8.17; Na, 12.13.

Found: N, 18.07; C, 28.67; H, 3.36; P, 8.51, Na 11.90 (by atomic absorption). λmax 252 nm, ε, 9600 (0.1M pH 7 phosphate).

Example 14

9-[1,3-Bis(phenoxyacetoxy)-2-propoxymethyl]guanine

A suspension of 273 mg (1.0 mmole) of 9-(1,3-dihydroxy-2-propoxymethyl)guanine monohydrate in 4 ml of dry dimethylformamide and 1.4 ml of dry pyridine was stirred under nitrogen with cooling in an ice bath as a solution of 522 µl (682 mg, 4 mmole) of phenoxyacetyl chloride in 1.6 ml of dimethylformamide was added dropwise by syringe through a septum over a period of 10 minutes. After the ice had melted, the mixture was allowed to warm gradually to room temperature. A pale yellow solution was obtained. After 15 hours the solution was concentrated under high vacuum with mild warming. The golden residual oil was chromatographed on a silica gel column (gradient elution from 98:2 $CH_2Cl_2$—MeOH to 92:8 $CH_2Cl_2$—MeOH). Fractions containing nearly pure product were combined and concentrated to give an oil which solidified on trituration with ether-acetone. Recrystallization from a small volume of acetonitrile gave 114 mg of white crystals, m.p. 114—116°. Structure and purity were confirmed by NMR and TLC (9:1 $CHCl_3$—MeOH). A second crop of 66 mg was obtained from the mother liquor.

Anal. ($C_{25}H_{25}N_5O_8$).

Calcd.: for 93.5% $C_{25}H_{25}H_5O_8 \cdot H_2O$ + 6.5% inorganic, C, 51.84; H, 4.70; N, 12.09.

Found: C, 51.94; H, 4.74; N, 11.99.

Example 15

9-(2,3-Dibenzoyloxy-1-propoxymethyl)guanine

A suspension of hydrated 9-(2,3-dihydroxy-1-propoxymethyl)guanine (1 mmole) in 4 ml of dry dimethylformamide and 1.4 ml of dry pyridine is stirred under nitrogen in an ice bath as a solution of benzoyl chloride (4 mmole) in 1.6 ml of dimethylformamide is added dropwise. The mixture is allowed to warm gradually to room temperature. After stirring overnight, the solution is concentrated under high vacuum. The residue is chromatographed on silica gel (elution with $CH_2Cl_2$—MeOH) to give the product. Structure and purity are confirmed by NMR and TLC (9:1 $CHCl_3$—MeOH).

Example 16

9-(1,3-Diisovaleryloxy-2-propoxymethyl)guanine

A suspension of hydrated 9-(1,3-dihydroxy-2-propoxymethyl)guanine (1 mmole) in 4 ml of dry dimethylformamide and 1.4 ml of dry pyridine is stirred under nitrogen in an ice bath as a solution of isovaleryl chloride (4 mmole) in 1.6 ml of dimethylformamide is added dropwise. After warming gradually to room temperature, the mixture is stirred overnight. The resulting solution is evaporated under high vacuum with mild warming. Chromatography of the residue on silica gel (elution with $CH_2Cl_2$—MeOH) gives the product. Structure and purity are confirmed by NMR and TLC (9:1 $CHCl_3$—MeOH).

Example 17

9-[1,3-Bis(phenylacetoxy)-2-propoxymethyl]guanine

The compound is prepared by reaction of hydrated 9-(1,3-dihydroxy-2-propoxymethyl)guanine (1 mmole) with phenylacetyl chloride (4 mmole) according to the procedure used for 9-[1,3-bis(phenoxyacetoxy)-2-propoxymethyl]guanine (Example 14). After chromatography the product is characterized by NMR and TLC (9:1 $CHCl_3$—MeOH).

Example 18

9-[1,3-Bis(10-undecenoyloxy)-2-propoxymethyl]guanine

The compound is prepared by reaction of hydrated 9-(1,3-dihydroxy-2-propoxymethyl)guanine (1 mmole) with 10-undecenoyl chloride (4 mmole) according to the method used for 9-[1,3-bis(phenoxy-acetoxy)-2-propoxymethyl]guanine (Example 14). The product is obtained after chromatography. Structure and purity are confirmed by NMR and TLC (9:1 $CHCl_3$—MeOH).

Example 19

9-[1,3-Bis(methoxyacetoxy)-2-propoxymethyl]guanine

Reaction of hydrated 9-(1,3-dihydroxy-2-propoxymethyl)guanine (1 mmole) with methoxyacetyl chloride (4 mmole) according to the method used for 9-[1,3-bis(phenoxyacetoxy)-2-propoxymethyl]-guanine (Example 14) gives, after chromatography, the desired product. Confirmation of structure and purity are obtained by NMR and TLC (9:1 $CHCl_3$—MeOH).

Example 20

9-[1,3-Bis(imidazol-1-ylcarbonyloxy)-2-propoxymethyl]guanine

A mixture of 55 mg (0.2 mmole) of 9-(1,3-dihydroxy-2-propoxymethyl)guanine monohydrate, 130 mg (0.8 mmole) of 1,1′-carbonyldiimidazole, and 2 ml of dry dimethylformamide was stirred under nitrogen at 95—100° for 1.5 hours, during which time a clear solution was obtained followed by precipitation of product. After cooling, the precipitate was collected on a filter and washed with some dimethylformamide and then with acetone to give 37 mg of white crystals, m.p. 252—253° dec. The NMR spectrum was in accord with the assigned structure.

Anal. ($C_{17}H_{17}N_9O_6$).

Calcd.: C, 46.05; H, 3.87; N, 28.43;

Found: C, 45.68; H, 3.90; N, 28.18.

Example 21

Comparative Solubilities in pH 7.2 Buffer at 25°

Solubilities were determined by suspending an excess amount of the compound in approximately 0.15 molar phosphate buffer (pH 7.2) and shaking overnight in a water bath at 25° to give a saturated solution. The concentration of the compound in the filtered solution was calculated on the basis of spectrophotometric measurements, i.e. comparison of the ultraviolet absorbance at the $\lambda_{max}$ for the saturated solution with the absorbance value observed for a known concentration of the compound. The results were summarized as follows:

| Compound | Solubility (mg/ml) |
|---|---|
| Acycloguanosine | 1.3—1.5 |
| Compound of Formula I | 3.6 |
| Compound of Formula II | 2.8 |

Example 22

Phosphorylation of Compounds of Formula I and II and of Acycloguanosine by Herpes virus-induced Thymidine Kinase

30 µg of compound of formula I dissolved in 30 µl of 50% dimethylsulfoxide (DMSO) were incubated in a final volume of 150 µl for 3 hours at 37° with 50 mM Tris-HCl buffer, pH 7.5, 2.5 mM adenosine triphosphate, 2.5 mM magnesium chloride, 7.5 mM phosphocreatine, 2 units of creatine kinase, 2 mM dithiothreitol, 2.5 mM sodium fluoride, 50 µg of bovine serum albumin and 0.0014 units of thymidine kinase, isolated from virus-infected HeLa cells (HSV1 virus), at a multiplicity of 10, (10 virus particles per cell), harvested 8 hours post infection) by the method of CHENG & OSTRANDER (Journal of Biological Chemistry, 1976, vol. *251,* p 2605).

Two similar mixtures, one containing 30 µg of compound of Formula II and the other 30 µg of acycloguanosine, both in 50% DMSO were similarly treated.

A fourth mixture, similar to the above but containing only 30 µl of 50% DMSO and no anti-viral compound was also treated similarly as a control.

At the end of the 3 hour incubation period, 10 µl samples of each of the mixtures were analyzed by HPLC using an AX—10 column and a potassium phosphate ($KH_2PO_4$) gradient elution (0.01 to 1.0 M). The amount of the monophosphate derivative of each anti-viral compound was estimated by integration of the area under the respective chromatographic peaks. The results indicated that 16% of acycloguanosine was converted to the monophosphate derivative while 90% of compound of formula I and 95% of compound of formula II were converted to the respective monophosphates under the same conditions.

To the rest of the incubation mixtures were now added 0.04 units of guanosine monophosphate kinase and 20 µl of an extract of HSV1-infected HeLa cells [The cells were infected with the virus at a multiplicity of

10 and harvested 8 hours later; they were suspended in a solution containing 0.35 M $KH_2PO_4$, pH 7.5, 0.5 mM dithiothreitol, 0.2% polyoxyethylene(9)octylphenol (Nonidet P—40), 14% glycerol at 50 mg/ml and after 30 minutes at 4° were centrifuged at 100,000 g; the supernatant liquid was the crude extract.] Incubation was continued at 30° for 4 more hours, after which samples were analyzed by HPLC and the amount of the triphosphate derivatives of each compound determined by integration of the area under the respective chromatographic peak. The results indicated that 31% of acycloguanosine was converted to the triphosphate under these conditions as compared to a conversion of 55% of compound of formula I and 93% of compound of formula II to the respective triphosphate derivatives.

Since phosphorylation is presumed to be a prerequisite for the anti-viral activity of these compounds, the higher rate of phosphorylation of compounds of formula I and II to the monophosphate and triphosphate derivatives represents a considerable improvement over acycloguanosine.

Example 23

Enzymatic Preparation of the Acyclic Monophosphate of Compound of Formula II

The compound of formula II (25 mg) was incubated at 37° in a mixture containing: 50 mM potassium phosphate buffer at pH 6.5; bovine serum albumin, 1 mg/ml; adenosine triphosphate, 5 mM; magnesium chloride, 5 mM; dithiothreitol, 1 mM; phosphocreatine, 1 mM; creatine kinase, 12.5 units/ml; sodium fluoride, 2.5 mM; and 500 units of purified HSV1-induced thymidine kinase, in a total volume of 10 ml. The progress of the reaction was monitored by high performance liquid chromatography (HPLC). When 35% of compound II had been converted to the monophosphate, the reaction was terminated. The product was purified by HPLC chromatography on a preparative anion exchange column (AX—10, Varian) and desalted by chromatography on diethylaminoethyl cellulose (DEAE) with triethylammonium carbonate pH 7.6 as the eluting solvent. Freeze-drying of the solvent from the pooled fractions containing the product yielded 8 mg of compound of formula II monophosphate, the purity of which was confirmed by analytical HPLC.

Example 24

Enzymatic Preparation of the Diphosphate of Compound of Formula II

The compound of formula II (20 mg) was incubated at 37° in a 10 ml mixture containing: 50 mM potassium phosphate buffer at pH 6.5; bovine serum albumin, 1 mg/ml; adenosine triphosphate, 5 mM; magnesium chloride, 5 mM; dithiothreitol, 1 mM; phosphocreatine 1 mM; creatin kinase, 12.5 units/ml; sodium fluoride, 2.5 mM; 500 units of purified HSV1-induced thymidine kinase and 100 µg of guanosine monophosphate kinase from hog brain. The progress of the reaction was monitored by high performance liquid chromatography (HPLC). The incubation was continued for 4 hours at 37° and 20 hours at 30°. The pyrophosphate product was purified by HPLC chromatography on a preparative anion exchange column (AX—10, Varian) and desalted by chromatography on diethylaminoethyl cellulose (DEAE) with triethylammonium carbonate, pH 7.6 as the eluting solvent. Freeze-drying of the solvent from the pooled fractions containing the product yielded 10 mg of compound of formula II diphosphate, the purity of which was confirmed by analytical HPLC.

Example 25

Enzymatic Conversion of the Diphosphate of Compound of Formula II to the Linear Triphosphate

The diphosphate of compound of formula II (5 ml), prepared as in Example 23, was incubated at 37° in a 5 ml mixture containing: Tris-acetate buffer, pH 7.6, mM; magnesium chloride, 3 mM; ethylenediamine tetraacetic acid (ETDA) 1 mM; potassium phosphate, pH 7.5, 30 mM; pyruvate, 5 mM; glyceraldehyde phosphate, 30 mM; lactic dehydrogenase, 150 µg; glyceraldehyde phosphate dehydrogenase, 150 µg; 3-phosphoglycerate, kinase, 150 µg; and nicotinamide adenine dinucleotide ($NAD^+$), 15 mM. The progress of the reaction was monitored by high performance liquid chromatography (HPLC). Incubation was continued for 4 hours at 37° and 20 hours at 30°. The product was isolated by HPLC chromatography on an anion exchange column (AX—10, Varian) and desalted by chromatography on diethylaminoethyl cellulose (DEAE), with triethylammonium carbonate, pH 7.6 as the eluting solvent. Freeze-drying of the pooled fractions containing the product yielded 4 mg of the triphosphate of compound of formula II, the purity of which was confirmed by analytical HPLC.

Example 26

Enzymatic Preparation of the Triphosphate of Compound of Formula II

The compound of formula II (20 mg) was incubated at 37° in a 10 ml mixture containing: Tris-HCl, pH 7.5, 50 mM; magnesium chloride, 2.5 mM; adenosine-5'-triphosphate, 2.5 mM; bovine serum albumin, 500 mg/ml; dithiothreitol, 2 mM; phosphocreatine, 7 mM; creatine kinase, 12.5 units/ml; sodium fluoride, 2.5 mM; HSV1-induced thymidine kinase, 400 units; and guanosine monophosphate kinase, 80 µg. Incubation was continued for 4 hours at 37° and for 20 hours at 30°. The progress of the reaction was monitored by analytical high performance liquid chromatography. The product was isolated by HPLC chromatography on a preparative anion-exchange column (AX—10, Varian) and then rechromatographed on an analytical column, (Zorbax-$NH_2$) to remove contaminating ATP. The product was desalted by chromatography on diethylaminoethyl cellulose (DEAE) with triethylammonium carbonate pH 7.6 as the eluting solvent. Freeze-drying of the pooled fractions containing the product yielded 15 mg of the triphosphate derivative of compound of formula II, the purity of which was confirmed by analytical HPLC.

Example 27

Competition Between Thymidine and Acycloguanosine or Compound of Formula II for Phosphorylation by the Virus-Induced Thymidine Kinase

(1) 20 µg of acycloguanosine dissolved in 20 µl of 50% DMSO was incubated in a total volume of 120 µl (0.75 mM) with 80 mM Tris-HCl, pH 7.5, 4 mM adenosine triphosphate, 4 mM magnesium chloride, 1.7 mM dithiothreitol, 12.5 mM phosphocreatine, 5.0 mM sodium fluoride, 100 µg bovine serum albumin, 2.5 units of creatine kinase and 0.006 units of thymidine kinase, isolated from HSV1-infected HeLa cells (as per Example 22). Incubation was carried out at 37° for 2 hours and then continued at 30° for 18 hours.

(2) A second mixture containing the same ingredients as mixture No. 1 plus 2.5 mM thymidine was incubated in the same manner.

(3) A third mixture containing the same ingredients as mixture No. 1 but with 20 µg of compound of formula II replacing the acycloguanosine was incubated in the same manner.

(4) A fourth mixture containing the same ingredients as mixture No. 3 plus 2.5 mM thymidine was incubated in the same manner.

At the end of the incubation the amount of each anti-viral compound converted to the corresponding monophosphate derivative was determined after HPLC analysis by integration of the areas under the chromatographic peaks (column and elution conditions as in Example 22).

The percent of monophosphate present at the end of incubation in the four mixtures was as follows:

| Mixture No. | Compounds present | Percent monophosphate |
|---|---|---|
| 1 | Acycloguanosine | 27 |
| 2 | Acycloguanosine & Thymidine | 0 |
| 3 | Compound of Formula II | 93 |
| 4 | Compound of Formula II & Thymidine | 23 |

The results indicated that compound of formula II is phosphorylated by the viral thymidine kinase even in the presence of a large excess of thymidine whereas acycloguanosine was not phosphorylated at all under the same conditions. Since phosphorylation is a prerequisite for the anti-viral activity of these compounds and since thymidine is a normal constituent of the cells, the compound of formula II represents a significant improvement over acycloguanosine.

Example 28

Comparison of the Kinetic Parameters of Acycloguanosine and Compound of Formula II with Purified Viral Thymidine Kinase

A series of mixtures containing in a total volume of 100 µL: 22 µmoles $KPO_4$ buffer at pH 6.5; 0.3 µmoles $MgCl_2$; 0.5 µmoles of adenosine triphosphate; 100 µg of bovine serum albumin; 20 units of HSV1-induced thymidine kinase and varying amounts of either compound of formula II or acycloguanosine, labeled with radioactive carbon ($^{14}C$) at position 8 of the guanine ring were incubated for 15 minutes at 37°. At the end of this period, 80 µL aliquots from each tube were applied to circular filter papers (2.5 cm diameter) of diethylaminoethyl cellulose (Whatman DE81). Five minutes later, the filters were placed into a beaker with water and washed successively once with water, twice with 50% ethanol containing 0.5 mM guanosine, and once with absolute ethanol. They were then placed in scintillation vials, dried in a stream of air, and counted in a scintillation counter after addition of scintillation mixture (Aquasol 2, New England Nuclear). By this procedure, unphosphorylated compounds were washed away and only phosphorylated derivatives adhered to the DE81 filters; thus the radioactivity counted was a measure of the conversion of the substrates, compound of formula II or acycloguanosine, to their phosphorylated derivatives by the action of the viral thymidine kinase. Proper controls for background radioactivity were included in the assays and used to correct the results.

The number of moles of phosphorylated derivatives present in each assay tube at the end of the incubation period was calculated from the number of counts of radioactivity measured for each filter and the specific activity (counts per minute per mole) of each substrate in the assay mixtures. The data were plotted in a graph of reaction velocity versus substrate concentration. Figure 1 is a graph of the computer-generated theoretical curves best fitting the actual experimental data. The curve obtained in a similar experiment with thymidine as the substrate is included in Figure 1 for comparison.

The kinetic parameters $K_m$, $V_{max}$ and $V_{max}/K_m$ for the two substrates were computed from the same data.

The values obtained by averaging three separate experiments like the one described above were as follows:

| | Compound of formula II | Acycloguanosine |
|---|---|---|
| $K_m$ (μM) | 66 | 426 |
| $V_{max}$ (pmoles/min) | 280 | 61 |
| $V_{max}/K_m$ | 4.25 | 0.14 |

Inasmuch as the ratio $V_{max}/K_m$ is the most commonly used measure for comparing substrate efficiencies, the relative efficiencies of compound of formula II and acycloguanosine as substrates for the HSV1-induced thymidine kinase are 4.25 to 0.14 or 30 to 1.

Example 29

Comparison of the Kinetic Parameters of Acycloguanosine and Compound of Formula II with Purified Guanosine Monophosphate Kinase

A series of mixtures containing in a total volume of 700 μl: 70 μmoles Tris-acetate buffer at pH 7.6; 70 μmoles KCl; 7 μmoles $MgCl_2$; 2.8 μmoles ATP; 1.05 μmoles phosphoenolpyruvate; 175 μg bovine serum albumin; 0.15 μmoles reduced nicotinamide-adenine dinucleotide (NADH); 3 units lactic dehydrogenase; 1.5 units pyruvate kinase; and varying amounts of either acycloguanosine monophosphate or the monophosphate of compound of Formula II are incubated at 25° with guanosine monophosphate kinase from hog brain (Boehringer-Mannheim) in the cuvette of a Cary spectrophotometer recording the absorbance at 340 nm. In this coupled spectrophotometric assay the rate of phosphorylation of the monophosphate substrates to the corresponding diphosphates is calculated from the decrease in absorbance at 340 nm of the NADH. Since acycloguanosine monophosphate is a much poorer substrate for the kinase than compound of Formula II monophosphate, more enzyme is used in the case of acycloguanosine monophosphate (0.28 units) than in the case of compound of Formula II monophosphate (0.0056 units).

The initial velocities obtained in the above experiment are used to compute the kinetic parameters of the two substrates which are presented in the following table. The parameters obtained in a similar experiment for deoxyguanosine monophosphate are included for comparison:

| | Compound of Formula II Monophosphate | Acyclo-guanosine monophosphate | Deoxy-guanosine monophosphate |
|---|---|---|---|
| $K_m$ (μM) | 22 | 316 | 127 |
| $V_{max}$ (μg/min/mg) | 7.1 | 0.20 | 17.2 |
| $V_{max}/K_m$ | 0.32 | 0.00065 | 0.14 |

The relative efficiency of compound of Formula II monophosphate and acycloguanosine monophosphate as substrates for the enzyme which converts them to the respective diphosphates in 0.32/0.00065 or 492 to 1.

Example 30

Enzymatic Preparation of the Acyclic Monophosphate of Compound of Formula I

The compound of formula I (1 mg) was incubated at 37° in a mixture containing: 50 mM potassium phosphate buffer at pH 6.5; bovine serum albumin, 1 mg/ml; adenosine triphosphate, 5 mM; magnesium chloride, 5 mM; dithiothreitol, 1 mM; phosphocreatine 1 mM; creatine kinase, 12.5 units/ml; sodium fluoride, 2.5 mM; and 20 units of purified HSV1-induced thymidine kinase, in a total volume of 0.5 ml. The progress of the reaction was monitored by high performance liquid chromatography (HPLC). When 65% of the compound of formula I had been converted to the monophosphate, the reaction was terminated. The product was purified by HPLC chromatography on a preparative anion exchange column (AX—10, Varian) and desalted by chromatography on diethylaminoethyl cellulose (DEAE) with triethylammonium carbonate pH 7.6 as the eluting solvent. Freeze-drying of the solvent from the pooled fractions containing the product yielded 500 μg of compound I monophosphate, the purity of which was confirmed by analytical HPLC.

Example 31

Treatment of Virus Infections in Cell cultures *in Vitro*

Assays were performed in various cell culture systems to determine the minimum concentrations of

the compounds of Formula I, Formula II or acycloguanosine that were effective in preventing several different kinds of virus infections.

a. *Herpes simplex virus types 1 and 2:* The compounds of Formula I, Formula II or acycloguanosine required to totally suppress the development of viral cytopathology in 50% of rabbit kidney cell monolayers infected with 10 tissue culture infectious doses ($TCID_{50}$) of either virus are shown in the attached Table. All three compounds showed comparable activity.

b. *Varicella-Zoster virus:* Both the compound of Formula II and acycloguanosine were equally active against this herpesvirus as determined by a plaque-reduction assay using monolayers of human fetal diploid lung cells, MRC—5. The results are shown in the attached Table.

c. *Epstein-Barr virus (EBV):* Continuous treatment of EBV-infected umbilical cord cells (B lymphocytes) with 1—5 µg/ml of the compound of Formula II from the time of infection resulted in inhibition of the transformation of the normal lymphocytes into continuously growing lymphoblastoid cells. By contrast, between 10 and 100 µg/ml of acycloguanosine were required to show similar activity. The results are shown in the attached Table.

d. *Cytomegalovirus:* The compound of Formula II was effective in suppressing cytomegalovirus plaque formation on MRC—5 cell monolayers using 0.1 to 0.6 µg/ml. In order to obtain equivalent plaque suppression (50%) using acycloguanosine required 2.2—17.7 µg/ml. The calculated average relative potency of the compound of Formula II to acycloguanosine (95% CI) was 28.6. The results are shown in the attached Table.

Minimum Concentrations of Formula I, Formula II or Acycloguanosine
Active Against Hervesviruses in Cell Cultures

| | Minimum Effective Concentration (µg/ml) | | |
|---|---|---|---|
| Virus | Formula I | Formula II | Acycloguanosine |
| Herpes simplex type 1 (Strain Schooler) | 1—3[a] | 1—3[a] | 1—3[a] |
| Herpes simplex type 1 (Strain S) | ND | 1—3[a] | ND |
| Herpes simplex type 1 (Strain McIntyre) | ND | 3[a] | ND |
| Herpes simplex type 1 (Strain McKrae) | ND | 1—3[a] | ND |
| Herpes simplex type 2 (Strain Curtis) | 3—6[a] | 1—3[a] | 1—3[a] |
| Varicella-Zoster (Strain KMcC) | ND | 1—2[b] | 1—2[b] |
| Epstein-Barr ($B_{95-8}$) | ND | 1—5[c] | 10—100[c] |
| Cytomegalovirus (Towne Strain) | ND | 0.1—0.6[d] | 2.2—17.7[d] |

ND — Not done.
a — Tube dilution assay on primary rabbit kidney cell cultures.
b — Plaque reduction assay on human MRC—5 cell monolayers.
c — Human cord blood lymphocyte transformation assay.
d — Plaque reduction assay on human MRC—5 cell monolayers.

### Example 32
### Treatment of Herpes Simplex Virus Infection in Mice

Twenty gram ICR/Ha mice were injected intraperitoneally (ip) with 0.5 ml of a $10^{-5}$ dilution of a stock preparation of Herpes simplex virus type I (HSV—1), strain Schooler. This virus challenge infected each animal with approximately 100 $LD_{50}$. Starting immediately after virus infection and continuing twice daily for 4 days, each animal was injected subcutaneously in groups of 15 with: 500 µg, 125 µg or 31 µg of acycloguanosine; 500 µg, 125 µg, or 31 µg of the compound of formula I; 500 µg, 125 µg, or 31 µg of the compound of formula II; or placebo (physiological saline, pH 11.5). The placebo group was composed of 45 animals.

All compounds were solubilized in physiological saline, pH 11.5.

The mice were observed daily for 15 days at the same time each day and the day of death was recorded for each animal.

Statistical analyses (reference: Liddel, F.D.K., 1978, Evaluation of Survival in Challenge Experiments, Microbiol. Rev., 42: 237—249) were performed on survival times transformed by the negative exponential transformation:

$$f(t) = 1 - (0.1)^{t/T}$$

where t = number of days an animal survived
T = duration of trial (15 days)

A continuity correction was used to account for daily observation:

$$f_c(t) = 1/2\,[f(t) + f(t-1)]$$

Within each group, mice surviving through the trial period were assigned equally values of 0.9 and 1.0 to adjust for termination of the trial.

Average survival time per group was *calculated* from average corrected transformed survival times $[f_c(t)]$ as follows:

$$t\ avq = [T/\log(0.1)] \cdot [\log(1-f_c(t))]$$

The summarized results are shown in the following Table:

| Chemical Agent | Animal Treatment µg/dose | mg/kg/day | Percent Survival* | Avg. Survival Time (Days) |
|---|---|---|---|---|
| Acycloguanosine | 500 | 50 | 0 | 7.7 |
| | 125 | 12.5 | 6 | 6.2** |
| | 31 | 3.1 | 6 | 6.4** |
| Compound of Formula I | 500 | 50 | 60 | 11.8 |
| | 125 | 12.5 | 26 | 9.0 |
| | 31 | 3.1 | 0 | 6.3** |
| Compound of Formula II | 500 | 50 | 100 | 19.1 |
| | 125 | 12.5 | 73 | 14.1 |
| | 31 | 3.1 | 53 | 12.5 |
| Placebo | 0.1 ml | — | 6 | 6.2 |

* determined at 15 days
** values not statistically different from that of placebo treated animals (P>0.05)

Example 33
Treatment of Herpes Simplex Virus Infection in Mice

The experiment described in Example 32 was repeated, except that each animal was injected twice daily subcutaneously in groups of 15 with: 1000 µg, 500 µg or 125 µg of acycloguanosine; 500 µg, 125 µg or 31 µg of the compound of Formula I; 500 µg, 125 µg, 31 µg, 8 µg or 2 µg of the compound of Formula II; or placebo. The 1000 µg dose acycloguanosine treatment group and the 500 µg dose treatment group of the compound of Formula II were composed of 10 animals each. The summarized results are shown in the following Table:

| Chemical Agent | Animal Treatment | | Percent Survival* | Avg. Survival Time (days) |
|---|---|---|---|---|
| | µg/dose | mg/kg/day | | |
| Acycloguanosine | 1000 | 100 | 10 | 7.8 |
| | 500 | 50 | 0 | 7.8 |
| | 125 | 12.5 | 6 | 7.0** |
| Compound of | 500 | 50 | 36 | 10.5 |
| Formula I | 125 | 12.5 | 33 | 9.8 |
| | 31 | 3.1 | 6 | 7.2** |
| Compound of | 500 | 50 | 100 | 19.5 |
| Formula II | 125 | 12.5 | 93 | 17.2 |
| | 31 | 3.1 | 46 | 11.8 |
| | 8 | 0.8 | 40 | 9.8 |
| | 2 | 0.2 | 26 | 9.0 |
| Placebo | 0.1 ml | — | 0 | 6.3 |

* determined at 15 days
** values not statistically different from that of placebo treated animals ($P>0.05$)

Using combined results from Examples 32 and 33, the calculated average relative potencies of the compound of Formula I and the compound of Formula II to acycloguanosine (95% CI) were 9.2 and 287.0, respectively.

Example 34

The following is a summary of *in vitro* and *in vivo* antiviral activities of potassium 9-1,3-dihydroxy-2-propoxymethyl)guanine cyclic monophosphate against Herpes simplex virus Type 1 (HSV1) and Type 2 (HSV2).

*In Vitro Assays:*

*Method:* Confluent monolayers of primary rabbit kidney cell cultures were refed with maintenance medium containing serial dilutions of the test compounds and incubated overnight at 37°. At each dilution, four cultures were challenged with approximately 10 $TCID_{50}$ HSV1, four cultures were challneged with approximately 10 $TCID_{50}$ HSV2 and two cultures were left as toxicity controls. Cultures were reincubated at 37° and observed for viral induced cytopathology at days 5 and 7.

*Results:*

| Compound | Min. Effective Dose (µg/ml) Against HSV1 | HSV2 | Toxicity |
|---|---|---|---|
| Potassium 9-(1,3-dihydroxy-2-propoxymethyl)guanine | 12.5 | 50 | Not tox. at 100 |
| cyclic monophosphate | 25 | 100 | |

*In Vivo Assays:*

*Method:* Twenty gram ICR/Ha mice were infected with approximately 100 lethal doses ($100LD_{50}$) of HSV1 (Strain Schooler) by the intraperitoneal route. Groups of 10 infected animals were treated twice daily for four days starting immediately after infection by subcutaneous injection at final daily doses of 50, 12.5,

3.1, 0.8, 0.2, 0.05 and 0.0125 mg/kg of potassium 9-(1,3-dihydroxy-2-propoxymethyl)guanine cyclic monophosphate. The mice were observed daily for 15 days at the same time each day and the day of death recorded for each animal. Average survival times (days) and percent survival at 15 days are shown in the accompanying table.

| Compound | Animal Treatment mg/kg/day | Percent Survival Expt. 1 | Percent Survival Expt. 2 | Ave. Survival Time (days) Expt. 1 | Ave. Survival Time (days) Expt. 2 |
|---|---|---|---|---|---|
| Potassium 9-(1,3-dihydroxy-2-propoxymethyl)-guanine cyclic monophosphate | 50 | 100 | ND | 19.5 | ND |
| | 12.5 | 100 | 60 | 19.5 | 15.0 |
| | 3.1 | 20 | 50 | 10.7* | 11.5 |
| | 0.8 | 40 | 0 | 10.5* | 7.4* |
| | 0.2 | 70 | 30 | 13.5 | 8.3* |
| | 0.05 | ND | 0 | ND | 6.0* |
| | 0.0125 | ND | 10 | ND | 6.3* |
| Placebo | 0.1 ml | 20 | 10 | 7.8 | 6.4 |

* — Values not statistically different from that of placebo treated animals (P 0.05) calculated for survival times.

Conclusion: Potassium 9-(1,3-dihydroxy-2-propoxymethyl)guanine cyclic monophosphate as demonstrated significant antiviral activity against Herpes simplex viruses *in vitro* and *in vivo.*

Example 35
Treatment of Herpes Simplex Virus Infection in Mice:
Intraperitoneal *Herpes Simplex* Type 1 Infection, Oral Treatment

ICR/Ha mice were infected as described in Example 32. Groups of 10 infected animals were treated twice daily for 7 days by oral gavage at final daily doses of 100, 50, 12.5, 3.1 or 0.8 mg/kg of acycloguanosine, or 50, 12.5, 3.1, 0.8 or 0.2 mg/kg of the compound of Formula II starting immediately after infection. In addition, two groups of five uninfected animals were treated with either acycloguanosine or the compound of Formula II twice daily for 7 days by oral gavage at final daily doses of 50 mg/kg. These animals served as toxicity controls. The summarized results are shown in the following Table:

Oral Treatment of
Intraperitoneal Herpes Simplex Virus Type I Infection of Mice

| Chemical Agent | Animal Treatment µg/dose | mg/kg/day | Percent Survival* | Avg. Survival Time (days) |
|---|---|---|---|---|
| Acycloguanosine | 1000 | 100 | 60 | 11.4 |
| | 500 | 50 | 20 | 8.2 |
| | 125 | 12.5 | 0 | 6.6** |
| | 31 | 3.1 | 0 | 6.3** |
| The Compound | 500 | 50 | 100 | 16.9 |
| of Formula II | 125 | 12.5 | 80 | 13.9 |
| | 31 | 3.1 | 50 | 11.7 |
| | 8 | 0.8 | 20 | 8.5 |
| | 2 | 0.2 | 0 | 6.8** |
| Placebo | 0.1 | | 10 | 6.3 |

* determined at 13 days
** values not statistically different from that of placebo treated animals (P>0.05). Calculated for survival times only.

Treatment with the compound of Formula II resulted in statistically significant extension of survival time compared to placebo-treated animals at 50, 12.5, 3.1 and 0.8 mg/kg daily doses. Acycloguanosine treatment resulted in statistically significant extension of survival time compared to placebo-treated animals only at 100 and 50 mg/kg daily doses.

All animals treated with 50 mg/kg of the compound of Formula II survived the test; survival of the 50 and 12.5 mg/kg treatment groups was statistically significantly longer than the placebo-treated group. By contrast, none of the acycloguanosine-treated groups showed enhanced survival.

The relative potency of the compound of Formula II to acycloguanosine was 50.3, which was statistically significant.

There was no evidence of overt toxicity in either the acycloguanosine or the compound of Formula II treatment groups as measured by final weight of test animals.

Example 36

Treatment of Herpes Simplex Virus Infection in Mice:
Vaginal *Herpes Simplex* Virus Type 2 Infection, Oral Treatment

Thirty gram ICR/Ha female mice were infected with more than 10 $LD_{50}$ of *Herpes simplex* virus type 2 (Strain Curtis) by the intravaginal route. Groups of 10 animals were treated twice daily for ten days by oral gavage using acycloguanosine or the compound of Formula II at final daily doses of 50, 12.5, 3.1, 0.8 or 0.2 mg/kg starting immediately following infection. The average number of days to infection and the average days of survival were determined for each group and compared to an infected, placebo-treated group. The summarized results are shown in the following Table.

ORAL TREATMENT OF VAGINAL HERPES SIMPLEX VIRUS TYPE II INFECTION OF MICE

| Chemical Agent | Animal Treatment | | Percent Survival* | Avg. Survival Time (days) |
|---|---|---|---|---|
| | µg/dose | mg/kg/day | | |
| Acycloguanosine | 750 | 50 | 60 | 17.6 |
| | 188 | 12.5 | 40 | 14.1 |
| | 47 | 3.1 | 20 | 10.2** |
| | 12 | 0.8 | 10 | 10.1** |
| | 3 | 0.2 | 0 | 8.8** |
| The Compound of Formula II | 750 | 50 | 100 | 24.7 |
| | 188 | 12.5 | 100 | 24.7 |
| | 47 | 3.1 | 75 | 19.1 |
| | 12 | 0.8 | 40 | 15.1 |
| | 3 | 0.2 | 30 | 12.1** |
| Placebo | 0.1 ml | — | 0 | 9.3 |

* determined at 19 days
** values not statistically different from that of placebo treated animals (P>0.05). Calculated for survival times only.

All animals treated with the compound of Formula II at 50 mg or 12.5 mg/kg survived the test; survival rates of the 50, 12.5 and 3.1 mg/kg the compound of Formula II treatment groups were statistically significantly increased compared to the placebo-treated group. By contrast, only the 50 mg/kg acycloguanosine treatment group showed statistically significant enhanced survival.

The compound of Formula II at 50, 12.5, 3.1 and 0.8 mg/kg resulted in a statistically significant increase in survival time compared to placebo-treated infected animals. Acycloguanosine at 50 and 12.5 mg/kg was similarly effective.

The relative potency of the compound of Formula II to acycloguanosine as measured by survival was 28.1, which was statistically significant.

All animals treated with the compound of Formula II at 50 mg/kg remained free of signs of herpetic infection for the duration of the test. The compound of Formula II and acycloguanosine at both 50 mg/kg and 12.5 mg/kg resulted in statistically significant increases in the number of days to infection (development of vaginal lesions and/or paralysis) compared to placebo-treated animals.

The relative potency of the compound of Formula II to acycloguanosine as measured by time to infection was 4.14, which was statistically significant.

Example 37

Treatment of Herpes Simplex Virus Infection in Mice:
Orofacial Herpes Simplex Virus Type 1 Infection, Oral Treatment

Twenty gram HRS (hairless) mice were infected on the abraded orofacial area with *Herpes simplex* virus type 1 (Strain S). Groups each composed of 10 infected animals were treated by oral gavage twice daily for 7 days starting 3 hours after infection using final daily doses of 50, 12.5, 3.1 and 0.8 mg/kg for acycloguanosine and 50, 12.5, 3.1, 0.8 and 0.2 mg/kg for the compound of Formula II. At 7 days after initiation of infection, the extent of lesion development in the orofacial area was measured on a scale of 0 (no lesions) to 4 (massive lesions over the entire snout). Lesion incidence and average lesion scores are shown in the accompanying Table and in Figure 2.

The compound of Formula II treatment resulted in statistically significant protection at *all* concentrations used compared to placebo-treated infected animals when measured in terms of extent of lesion development. Acycloguanosine treatment resulted in statistically significant protection only at 50

and 12.5 mg/kg doses.

The compound of Formula II treatment resulted in statistically significant protection at 50 and 12.5 mg/kg doses compared to the incidence in placebo-treated infected animals when measured in terms of incidence of lesions. By contrast, acycloguanosine treatment resulted in statistically significant protection only at 50 mg/kg.

The relative potency of the compound of Formula II to acycloguanosine was 6.9, which was statistically significant.

| Chemical Agent | Animal Treatment | | Lesion Incidence | | Lesion Severity[a] |
|---|---|---|---|---|---|
| | μg/dose | mg/kg/day | Total | (%) | |
| Acycloguanosine | 500 | 50 | 1/7 | (14) | 0.14 |
| | 125 | 12.5 | 6/6 | (100)** | 2.67 |
| | 31 | 3.1 | 6/6 | (100)** | 3.25** |
| | 8 | 0.8 | 7/8 | (88)** | 3.11** |
| The Compound of Formula II | 500 | 50 | 1/10 | (10) | 0.05 |
| | 125 | 12.5 | 2/10 | (20) | 0.45 |
| | 31 | 3.1 | 6/9 | (67)** | 1.00 |
| | 8 | 0.8 | 7/7 | (100)** | 2.93 |
| | 2 | 0.2 | 7/7 | (100)** | 2.93 |
| Placebo | 0.1 ml | — | 10/10 | (100) | 3.95 |

[a] Lesion severity was measured on a scale of 0 (no lesions) to 4 (massive lesions over the entire orofacial area), with the average lesion score presented in this Table.

** Values not statistically different from that of placebo treated animals ($P>0.05$).

Lesion incidence and severity were determined seven days following orofacial infection.

## Example 38

### Treatment of *Herpes Simplex* Virus Infection in Mice: Orofacial Herpes simplex Virus Type 1 Infection, Therapeutic Oral Treatment

Twenty gram HRS (hairless) mice were infected on the abraded orofacial area with *Herpes simplex* virus type 1 (Strain S).

a. Groups of 10 infected animals were treated twice daily by oral gavage for up to 7 days with the compound of Formula II at 12.5 mg/kg/day, starting 3, 8, 12, 24, 48, 72, or 96 hours after infection.

b. In a second experiment, the therapeutic efficacy of acycloguanosine was evaluated in a similar manner. Groups of 10 infected animals were treated twice daily by oral gavage for up to 7 days with acycloguanosine at 12.5 mg/kg/day, starting at 3, 8, 12, 24, 48, 72, or 96 hours after infection.

At 7 days after initiation of infection, the extent of lesion development in the orofacial area was measured on a scale of 0 (no lesions) to 4 (massive lesions over the entire snout). Average lesion scores are shown in the accompanying Table.

Infected mice receiving the compound of Formula II starting as late as 72 hours after infection with HSV—1 showed statistically significant lower lesion scores than mice receiving placebo. By contrast, for acycloguanosine treatment only mice receiving treatment starting 3 hours after infection were statistically different in lesion score than the placebo group.

In addition, infected mice receiving the compound of Formula II starting at 8, 12 and 24 hours after infection had a statistically significant lower incidence of lesion development than mice receiving placebo. None of the acycloguanosine treated groups had a significantly lower incidence of lesions than the respective placebo treated animals.

Therapeutic Oral Treatment of Orofacial Herpes Simplex Virus Type I Infection of Mice

| Chemical Agent | Start of Oral[a] Treatment (Hours post infection) | Lesion Incidence Total | (%) | Lesion Severity[b] |
|---|---|---|---|---|
| Acycloguanosine 12.5 mg/kg/day | 3 | 10/10 | (100)** | 2.80 |
| | 8 | 10/10 | (100)** | 2.95** |
| | 12 | 9/10 | (90)** | 3.30** |
| | 24 | 9/10 | (90)** | 2.85** |
| | 48 | 8/10 | (80)** | 2.85** |
| | 72 | 9/10 | (90)** | 3.60** |
| | 96 | 7/7 | (100)** | 4.00** |
| Placebo | 3 | 9/9 | (100) | 3.35 |
| The Compound of Formula II 12.5 mg/kg/day | 3 | 6/10 | (60)** | 1.30 |
| | 8 | 3/10 | (30) | 0.55 |
| | 12 | 5/10 | (50) | 0.55 |
| | 24 | 4/10 | (40) | 0.40 |
| | 48 | 9/10 | (90)** | 1.50 |
| | 72 | 8/10 | (80)** | 2.55 |
| | 96 | 10/10 | (100)** | 3.85** |
| Placebo | 3 | 10/10 | (100) | 4.00 |

[a] Oral treatment was started at the indicated times after initiation of infection and continued with dosing twice each day for seven days.

Lesion incidence and severity were determined seven days following orofacial infection.

[b] Lesion severity was measured on a scale of 0 (no lesions) to 4 (massive lesions over the entire orofacial area), with the average lesion score presented in this table.

** Values not statistically different from that of placebo treated animals ($P > 0.05$).

Methods of Preparing Phosphate Derivatives of Compounds of Formula I and II

The mono- and polyphosphate derivatives of compounds of formulas I and II can be prepared chemically by reacting a compound of formula I or II with a phosphorylating agent such as phosphoryl chloride in a suitable aprotic solvent such as triethyl phosphate and treating the resulting intermediate with water or base. The major product of this reaction is the cyclic phosphate of compound I or II, but acyclic mono- and di-phosphates are also produced. The product ratios can be varied by changes in the amounts of the reactants or the length and temperature of treatment.

It is also convenient to isolate the phosphorylated intermediate by precipitation with a nonpolar hydrocarbon solvent and quench with an alcohol, particularly with an alcohol of 1 to 8 carbon atoms. The product of this reaction can be either an alkyl phosphotriester or an alkyl phosphodiester derivative depending upon wether a basic aqueous treatment is employed or not.

The phosphorylated derivatives of the compounds of formulas I and II [namely the mono-, linear di-(pyrophosphat), or linear tri-phosphate] can also be prepared enzymatically by treatment of the compound of formulas I or II with HSVI thymidine kinase (to produce the monophosphate), additionally with guanosine monophosphate kinase (to produce the pyrophosphate), and additionally with 3-phosphoglycerate kinase (to prepare the triphosphate).

**Claims**

1. A compound of the formula:

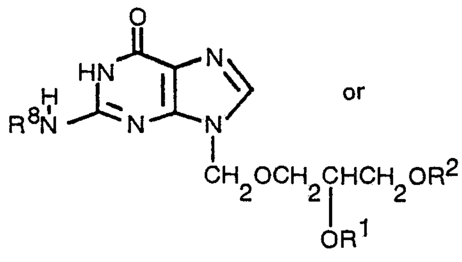

or

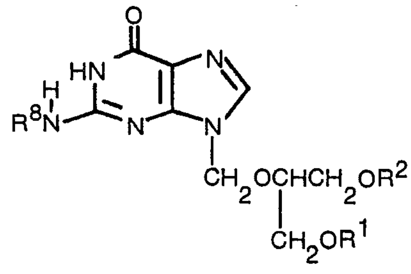

I II

and the pharmaceutically acceptable salts thereof, wherein $R^1$ and $R^2$ are independently H,

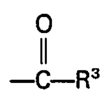

or

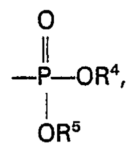

or $OR^1$ and $OR^2$ together are

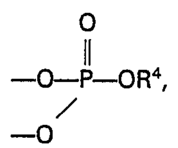

wherein $R^3$ is H, alkyl of 1—20 carbon atoms, preferably 1—10 carbon atoms, which may be straight chain or branched, saturated or mono- or polyunsaturated, phenyl, optionally substituted by halogen or $C_{1-4}$ alkyl, pyridyl, piperidyl, furyl, imidazolyl, tetrahydrofuryl or thienyl, $C_{1-4}$ alky, substituted by phenyl, alkoxyalkyl, wherein both the alkoxy and alkyl groups contain 1—4 carbon atoms, or $C_{1-4}$ alkyl substituted by phenoxy; $R^4$ and $R^5$ are independently H, a pharmaceutically acceptable cation, alkyl of 1 to 8 carbon atoms which may be straight chain or branched, phenyl optionally substituted by halogen or $C_{1-4}$ alkyl, phosphate or pyrophosphate, and $R^8$ is H or

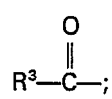

with the proviso that $R^1$, $R^2$ and $R^8$ do not simultaneously represent H in formula II and that $R^8$ is not acyl when $R^1$ and $R^2$ are H.

2. A compound of claim 1 wherin $OR^1$ and $OR^2$ together are

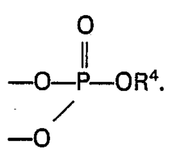

3. A compoound of claim 1 or 2 having the formula

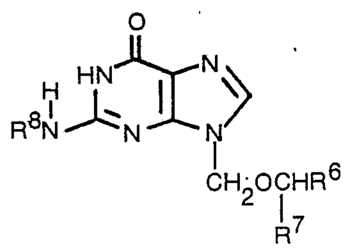

wherein $R^6$ is $—CH_2OH$ or

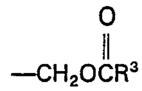

and $R^7$ is

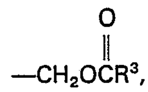

, or

wherein $R^6$ is

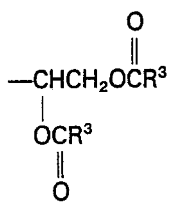

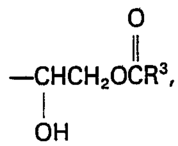

, or

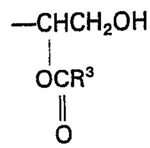

and $R^7$ is H and $R^8$ and $R^9$ as defined in claim 1.

4. A method of preparing a compound of claim 1 of the formula I, wherein $R^1$, $R^2$ and $R^8$ are each H which comprises reacting, at elevated temperature under vacuum in the presence of an acidic catalyst, a compound of the formula V

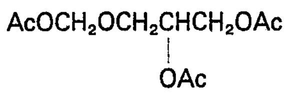

(V)

wherein Ac is acetyl, with a compound of the formula VII

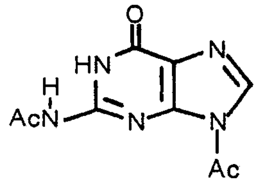

(VII)

wherein Ac is acetyl, to form a compound of the formula I and optionally deacylating a compound of the formula

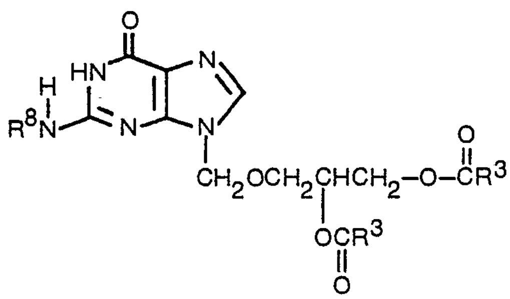

wherein $R^8$ is H or

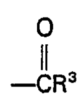

and $R^3$ is as defined in claim 1.

5. A method of preparing a compound of claim 1 of the formula II, wherein $R^1$, $R^2$ and $R^8$ are each H which comprises reacting at elevated temperature under vacuum in the presence of an acidic catalyst, a compound of the formula VI

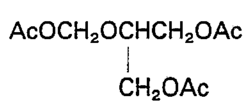

(VI)

wherein Ac is acetyl, with a compound of the formula VII as defined in claim 4, to form a compound of the

formula I and optionally deacylating a compound of the formula

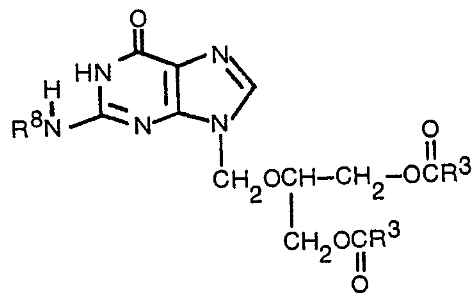

wherein $R^8$ is H or

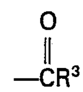

and $R^3$ is as defined in claim 1.

6. A method of preparing a compound of claim 3 which comprises reacting a compound of formula I or II of claim 1 wherein $R^1$, $R^2$ and $R^8$ are each H with an acylating compound of the formula

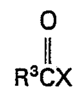

wherein X is a carboxyl activating group whereby a monoacylated derivative is formed when the reaction is allowed to proceed until 1 equivalent of the acylating compound is reacted with the compound of formula I or II, or whereby a diacylated derivative is formed when the reaction is allowed to proceed until 2 equivalents of the acylating compound are reacted with the compound of formula I or II or whereby a triacylated derivative is formed when 3 equivalents of the acylating agent are reacted with the compound of formula I or II.

7. A method according to claim 6 wherein the carboxyl activating group is halide, acyloxy, 1-benzotriazolyloxy, or N-succinimidyloxy.

8. A method of preparing a compound of claim 1 wherein $R^1$ and $R^2$ are H, or

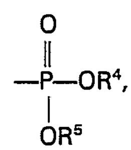

or $OR^1$ and $OR^2$ together are

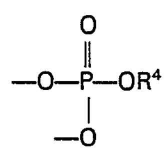

and $R^8$ is H, which comprises reacting a compound of formula I or II of claim 1 wherein $R^1$, $R^2$ and $R^8$ are each H with a phosphorylating agent and treating the resultant intermediate with water or an alkanol of 1—8 carbon atoms and isolating at least one of the resultant acyclic or cyclic phosphate derivatives.

9. A method according to claim 8 wherein the phosphorylating agent is $POX_3$ wherein X is halogen.

10. A method of preparing a compound of claim 1 wherein one of $R^1$ and $R^2$ is H and the other is

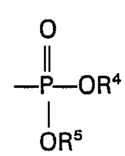

and $R^8$ is H and $R^4$ is as defined in claim 1 and $R^5$ is either H or a pharmaceutically acceptable cation which comprises reacting a compound of formula I or II, in which $OR^1$ and $OR^2$ together are

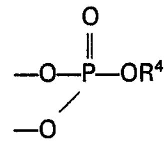

with an aqueous alkali metal hydroxide.

11. A method of preparing a monophosphate derivative of a compound of formula I or II of claim 1 wherein $R^1$, $R^2$ and $R^8$ are each H which comprises incubating said compound of formula I or II in the presence of HSV1 thymidine kinase and adenosine triphosphate.

12. A method for preparing a pyrophosphate derivative of a compound of formula I or II of claim 1 wherein $R^1$, $R^2$ and $R^8$ are each H which comprises incubating said compound fo formula I or II in the presence of HSV1 thymidine kinase, adenosine triphosphate and guanosine monophosphate kinase.

13. A method for preparing a linear triphosphate derivative of a compound of formula I or II of claim 1 wherein $R^1$, $R^2$ and $R^8$ are each H which comprises incubating said compound of formula I or II in the presence of HSV1 tymidine kinase, adenosine triphosphate, guanosine monophosphate kinase and extract of HSV1-infected cells.

14. A method for preparing a linear triphosphate derivative of a compound of formula I or II of claim 1 wherein $R^1$, $R^2$ and $R^8$ are each H which comprises incubating a compound of formula I or II wherein one of $R^1$ or $R^2$ is

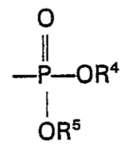

and the other is H and $R^4$ is phosphate, and $R^8$ is H, with 3′-phosphoglycerate kinase, 3-phosphoglyceraldehyde dehydrogenase and 3-phosphogylceraldehyde.

15. An intermediate for a compound of formula 1 of claim 1 of the formula

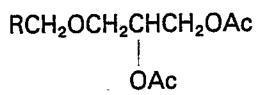

wherein R is AcO or bormine, chlorine or ioine, and Ac is acetyl.

16. An intermediate for a compound of formula II of claim 1 of the formula

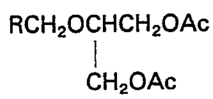

wherein R is AcO or bromine, chlorine or iodine, and Ac is acetyl.

17. A composition for treating a herpes virus infection in a mammalian species, which contains a compound of the formula I or II

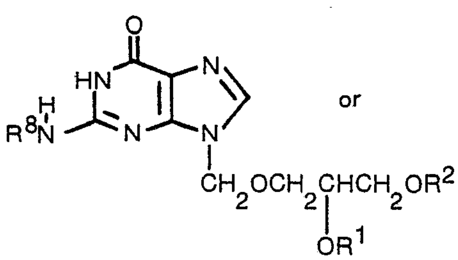

or

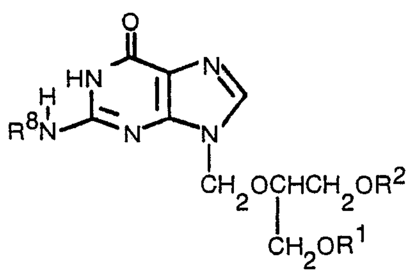

I II

and/or the pharmaceutically acceptable salts thereof, wherein $R^1$ and $R^2$ are independently H,

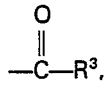

$R^3$ being defined as in claim 1,

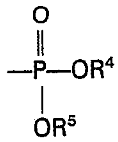

or $OR^1$ and $OR^2$ together are

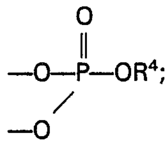

$R^4$ and $R^5$ are independently H, a pharmaceutically acceptable cation, alkyl of 1 to 8 carbon atoms which may be straight chain or branched, phenyl optionally substituted by halogen or $C_{1-4}$ alkyl, phosphate or pyrophosphate, and $R^8$ is H or acyl, with the proviso that $R^1$, $R^2$ and $R^8$ do not simulataneously represent H in formula II in a quantity effective to impart an anti-herpes virus effect.

18. A composition for treating a herpes virus infection in a mammalian or avian species, which contains a compound of the claim 2, in a quantity effective to impart an anti-herpes virus effect.

19. A composition for treating a herpes virus infection in a mammalian or avian species, which contains a compound of claim 3 in a quantity effective to impart an anti-herpes virus effect.

20. A composition according to one of the claims 17 to 19, for administering the compound at from about 0.01 to about 200 mg/kg.

21. A composition according to one of the claims 17 to 19, for topically adminstering the compound at a dosage level of from about 0.1% to about 5% by weight.

22. A composition according to claim 21 wherein the dosage level is from about 0.25% to aobut 3% by weight.

23. A composition according to one of the claims 17 to 19, for orally or parenterally administering the compound, at a dosage level of from 0.8 to about 100 mg/kg.

24. A composition according to claim 23 wherein the dosage level is from about 5 to about 50 mg/kg.

**Patentansprüche**

1. Verbindung der Formel

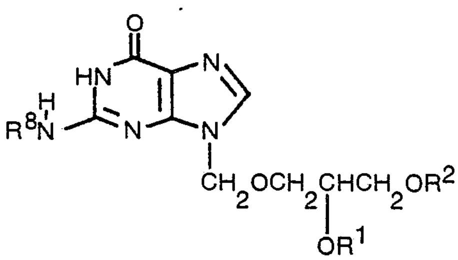

I

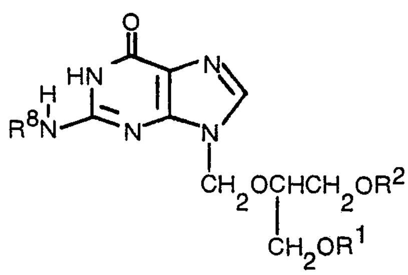

II

und pharmazeutisch annehmbare Salze davon, worin $R^1$ und $R^2$ unabhängig H,

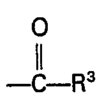

oder

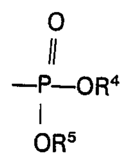

sind oder $OR^1$ und $OR^2$ zusammen

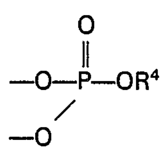

sind, worin $R^3$ H, Alkyl mit 1 bis 20 Kohlenstoffatomen, vorzugsweise 1 bis 10 Kohlenstoffatomen, das geradkettig oder verzweigt, gesättigt oder mono- oder polyungesättigt sein kann, gegebenenfalls durch Halogen oder $C_{1-4}$-Alkyl substituiertes Phenyl, Pyridyl, Piperidyl, Furyl, Imidazolyl, Tetrahydrofuryl oder Thienyl, durch Phenyl, Alkoxyalkyl substituiertes $C_{1-4}$-Alkyl, worin sowohl die Alkoxy- als auch die Alkylgruppen 1 bis 4 Kohlenstoffatome enthalten, oder durch Phenoxy substituiertes $C_{1-4}$-Alkyl ist; $R^4$ und $R^5$ unabhängig H, ein pharmazeutisch annehmbares Kation, Alkyl mit 1 bis 8 Kohlenstoffatomen, das geradkettig oder verzweigt sein kann, gegebenenfalls durch Halogen oder $C_{1-4}$-Alkyl substituiertes Phenyl, Phosphat oder Pyrophosphate sind, und $R^8$ H oder

$$R^3{-}\overset{\overset{\displaystyle O}{\|}}{C}{-} \quad \text{ist,}$$

mit der Massgabe, dass $R^1$, $R^2$ und $R^8$ in der Formel II nicht gleichzeitig H darstellen und dass $R^8$ nicht Acyl ist, wenn $R^1$ und $R^2$ H sind.

2. Verbindung nach Anspruch 1, worin $OR^1$ und $OR^2$ zusammen

$$\begin{array}{l} -O-\overset{\overset{\displaystyle O}{\|}}{P}-OR^4 \\ \quad\;\, / \\ -O \end{array} \quad \text{sind.}$$

3. Verbindung nach Anspruch 1 oder 2 mit der Formel

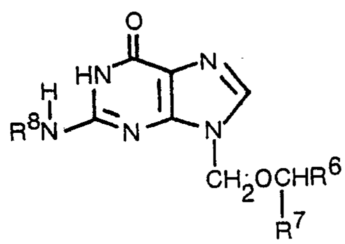

worin $R^6$ $-CH_2OH$ oder

$$-CH_2O\overset{\overset{\displaystyle O}{\|}}{C}R^3 \quad \text{ist und } R^7 \quad -CH_2O\overset{\overset{\displaystyle O}{\|}}{C}R^3$$

ist, oder worin $R^6$

$$\underset{\underset{\underset{\displaystyle O}{\|}}{OCR^3}}{\underset{|}{-CH}}CH_2O\overset{\overset{\displaystyle O}{\|}}{C}R^3, \quad \underset{\underset{\displaystyle OH}{|}}{-CH}CH_2O\overset{\overset{\displaystyle O}{\|}}{C}R^3 \quad \text{oder} \quad \underset{\underset{\underset{\displaystyle O}{\|}}{OCR^3}}{\underset{|}{-CH}}CH_2OH$$

ist und $R^7$ H ist und $R^8$ und $R^3$ wie in Anspruch 1 definiert sind.

4. Verfahren zur Herstellung einer Verbindung der Formel I nach Anspruch 1, worin $R^1$, $R^2$ und $R^8$ jeweils H darstellen, durch Reagieren, bei erhöhter Temperatur unter Vakuum in Gegenwart eines sauren Katalysators, einer Verbindung der Formel V

$$AcOCH_2OCH_2\underset{\underset{\displaystyle OAc}{|}}{C}HCH_2OAc \qquad (V)$$

worin Ac Acetyl ist, mit einer Verbindung der Formel VII

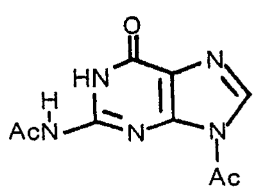

(VII)

worin Ac Acetyle ist, unter Bildung einer Verbindung der Formel I und gegebenfalls Deacylieren einer Verbindung der Formel

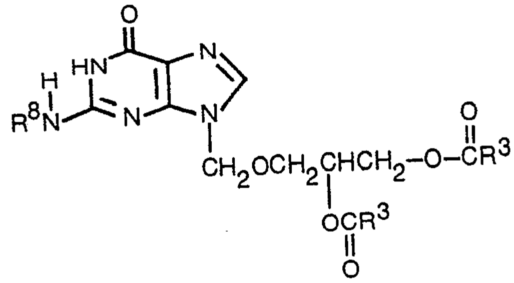

worin $R^8$ H oder

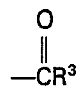

ist und $R^3$ wie in Anspruch 1 definiert ist.

5. Verfahren zur Herstellung einer Verbindung der Formel II nach Anspruch 1, worin $R^1$, $R^2$ und $R^8$ jeweils H sind, durch Reagieren, bei erhöhter Temperature unter Vakuum in Gegenwart eines sauren Katalysators, einer Verbindung der Formel VI

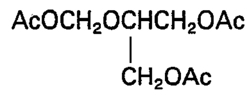
(VI)

worin Ac Actyl ist, mit einer Verbindung der Formel VII, wie in Anspruch 4 definiert, unter Bildung einer Verbindung der Formel I und gegebenenfalls Deacylieren einer Verbindung der Formel

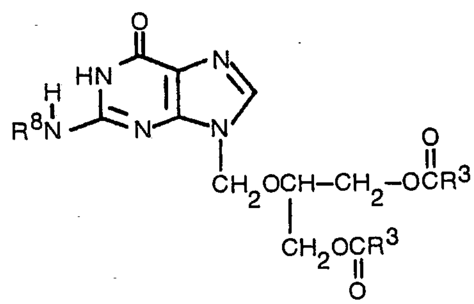

worin $R^8$ H oder

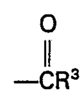

ist und $R^3$ wie in Anspruch 1 definiert ist.

6. Verfahren zur Herstellung einer Verbindung nach Anspruch 3 durch Reagieren einer Verbindung der Formel I oder II nach Anspruch 1, woring $R^1$, $R^2$ und $R^8$ jeweils H sind, mit einer acylierenden Verbindung der Formel

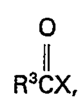

worin X eine Carboxyl-aktivierende Gruppe ist, wodurch ein monoacyliertes Derviat gebildet wird, wenn man die Reaktion fortschreiten läßt, bis 1 Äquivalent der acylierenden Verbindung mit der Verbindung der Formel I oder II reagiert hat, oder wodurch ein diacyliertes Derivat gebildet wird, wenn man die Reaktion fortschreiten läßt, bis 2 Äquivalente der acylierenden Verbindung mit der Verbindung der Formel I oder II reagiert haben, oder wodurch ein triacyliertes Derivat gebildet wird, wenn 3 Äquivalente des Acylierungsmittels mit der Verbindung der Formel I oder II reagiert werden.

7. Verfahren nach Anspruch 6, worin die Carboxyl-aktivierende Gruppe Halogen, Acyloxy, 1-Benzotriazolyloxy oder N-Succinimidyloxy ist.

8. Verfahren zur Herstellung einer Verbindung nach Anspruch 1, worin $R^1$ und $R^2$ H oder

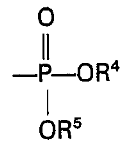

sind oder $OR^1$ und $OR^2$ zusammen

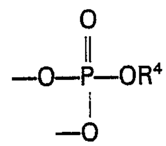

sind und $R^8$ H ist, durch Reagieren einer Verbindung der Formel I oder II nach Anspruch 1, worin $R^1$, $R^2$ und $R^8$ jeweils H sind, mit einem Phosphorylierungsmittel und Behandeln des resultierenden Zwischenprodukts mit Wasser oder einem Alkanol mit 1 bis 8 Kohlenstoffatomen und Isolieren von wenigstens einem der resultierenden acyclischen oder cyclischen Phosphatderivate.

9. Verfahren nach Anspruch 8, worin das Phosphorylierungsmittel $POX_3$ ist, worin X Halogen ist.

10. Verfahren zur Herstellung einer Verbindung nach Anspruch 1, worin eines aus $R^1$ und $R^2$ H ist und das andere

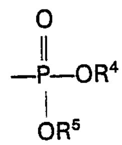

ist und $R^8$ H ist und $R^4$ wie in Anspruch definiert ist und $R^5$ entweder H oder ein pharmazeutisch annehmbares Kation ist, durch Reagieren einer Verbindung der Formel I oder II, worin $OR^1$ und $OR^2$ zusammen

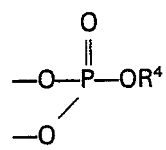

sind, mit einem wässrigen Alkalimetallhydroxid.

11. Verfahren zur Herstellung eines Monophosphatderivats einer Verbindung der Formel I oder II nach Anspruch 1, worin $R^1$, $R^2$ und $R^8$ jeweils H sind, durch Inkubieren dieser Verbindung der Formel I oder II in Gegenwart von HSV1-Thymidinkinase und Adenosintriphosphat.

12. Verfahren zur Herstellung eines Pyrophosphatderivats einer Verbindung der Formel I oder II nach Anspruch 1, worin $R^1$, $R^2$ und $R^8$ jeweils H sind, durch Inkubieren dieser Verbindung der Formel I oder II in Gegenwart von HSV1-Thymidinkinase, Adenosintriphosphat und Guanosinmonophosphatkinase.

13. Verfahren zur Herstellung eines linearen Triphosphatderivats einer Verbindung der Formel I oder II nach Anspruch 1, worin $R^1$, $R^2$ und $R^8$ jeweils H sind, durch Inkubieren dieser Verbindung der Formel I oder II in Gegenwart von HSV1-Thymidinkinase, Adenosintriphosphat, Guanosinmonophosphatkinase und Extrakt von HSV1-infizierten Zellen.

14. Verfahren zur Herstellung eines linearen Triphosphatderivats einer Verbindung der Formel I und II nach Anspruch 1, worin $R^1$, $R^2$ und $R^8$ jeweils H sind, durch Inkubieren einer Verbindung der Formel I oder II, worin eines aus $R^1$ oder $R^2$

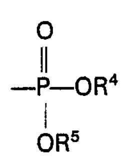

und $R^4$ Phosphat ist und $R^8$ H ist, mit 3'-Phosphoglyceratkinase, 3-Phosphogylceraldehyddehydrogenase und 3-Phosphoglyceraldehyd.

15. Zwischenprodukt für eine Verbindung der Formel I nach Anspruch 1 der Formel

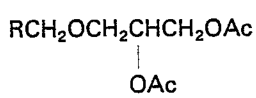

worin R AcO oder Brom, Chlor oder Jod ist und Ac Acetyl ist.

16. Zwischenprodukt für eine Verbindung der Formel II nach Anspruch 1 der Formel

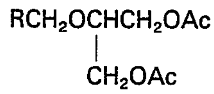

worin R AcO oder Brom, Chlor oder Jod is und Ac Acetyl ist.

17. Zusammensetzung zur Behandlung einer Herpesvirusinfektion in Säugetieren oder Vögeln, welche eine Verbindung der Formel I oder II

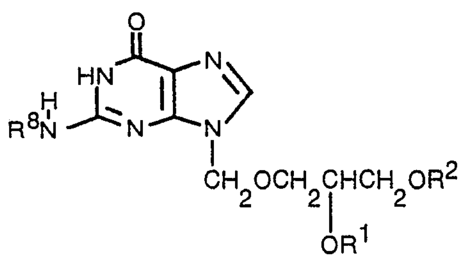

I

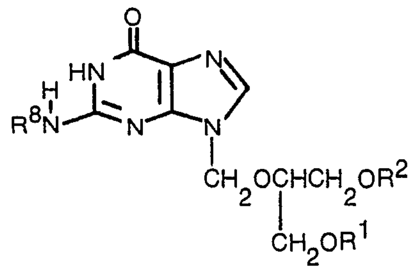

II

enthält und/oder pharmazeutisch annehmbare Salze davon, worin $R^1$ und $R^2$ unabhängig H,

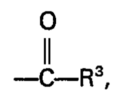

wobei $R^3$ wie in Anspruch 1 definiert ist,

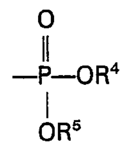

sind oder $OR^1$ und $OR^2$ zusammen

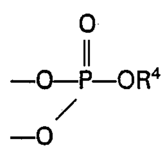

sind; $R^4$ und $R^5$ unabhängig H, ein pharmazeutisch annehmbares Kation, Alkyl mit 1 bis 8 Kohlenstoffatomen, des geradkettig oder verzweigt sein kann, gegebenenfalls durch Halogen oder $C_{1-4}$-Alkyl substitutiertes Phenyl, Phosphat oder Pyrophosphat sind und $R^8$ H oder Acyl ist, mit der Maßgabe, daß $R^1$, $R^2$ und $R^8$ in Formel II nicht gleichzeitig H darstellen, in einer zur Verleihung eines Antiherpes-Viruseffekts wirksamen Menge enthält.

18. Zusammensetzung zur Behandlung einer Herpesvirusinfektion in Säugetieren oder Vögeln, welche eine Verbindung nach Anspruch 2 in einer zur Verleihung eines Antiherpes-Viruseffekts wirksamen Menge enthält.

19. Zusammensetzung zur Behandlung einer Herpesvirusinfektion in Säugetieren oder Vögeln, welche eine Verbindung nach Anspruch 3 in einer zur Verleihung eines Antiherpes-Viruseffekts wirksamen Menge enthält.

20. Zusammensetzung nach einem der Ansprüche 17 bis 19 zur Verabreichung der Verbindung mit etwa 0,01 bis etwa 200 mg/kg.

21. Zusammensetzung nach einem der Ansrüche 17 bis 19 zur örtlichen Verabreichung der Verbindung in einer Dosis von etwa 0,1 bis etwa 5 Gew.-%.

22. Zusammensetzung nach Anspruch 21, worin die Dosis etwa 0,25 bis etwa 3 Gew.-% beträgt.

23. Zusammensetzung nach einem der Ansprüche 17 bis 19 zur oralen oder parenteralen Verabreichung der Verbindung in einer Dosis von 0,8 bis etwa 100 mg/kg.

24. Zusammensetzung nach Anspruch 23, worin die Dosis etwa 5 bis etwa 50 mg/kg beträgt.

**Revendications**

1. Une composé de formule:

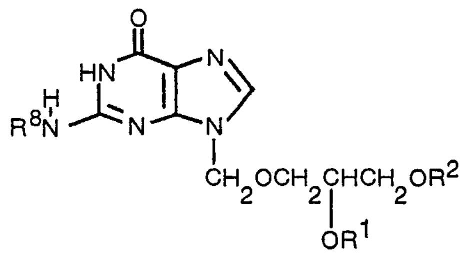 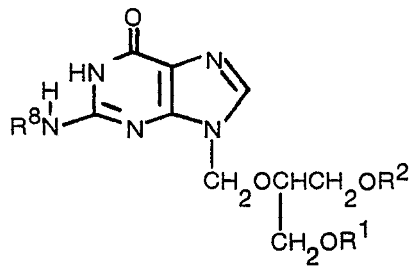

I II

et les sels correspondants convenant en pharmacie, où $R^1$ et $R^2$ sont indépendamment H,

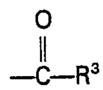 ou 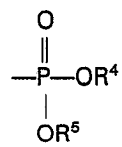

ou $OR^1$ et $OR^2$ ensemble sont

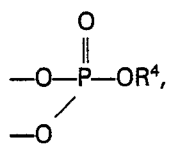

où $R^3$ et H, un alkyle de 1 à 20 atomes de carbone, de préférence de 1 à 10 atomes de carbone, qui peut être à chaine droite ou ramifiée, saturé ou mono- ou polyinsaturé, un phényle, facultativement substitué par un halogène ou un alkyle en $C_{1-4}$, un pyridyle, un pipéridyle, un furyle, un imidazolyle, un tétrahydrofuryle ou un thiényle, un alkyle en $C_{1-4}$, substitué par un phényle, un alcoxyalkyle dans lequel les groupes alcoxy et alkyle contiennent 1 à 4 atomes de carbone, ou un alkyle en $C_{1-4}$ substitué par un phénoxy; $R^4$ et $R^5$ sont indépendamment H, un cation convenant en pharmacie, un alkyle de 1 à 8 atomes de carbone qui peut être à chaîne droite ou ramifée, un phényle facultativement substitué par un halogène ou un alkyle en $C_{1-4}$, un phosphate ou un pyrophosphate, et $R^8$ est H ou

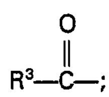

sous réserve que $R^1$, $R^2$ et $R^8$ ne ne représentent pas simultanément H dans la formule II et que $R^8$ ne soit pas un acyle lorsque $R^1$ et $R^2$ sont H.

2. Un composé selon la revendication 1 où $OR^1$ et $OR^2$ sont ensemble

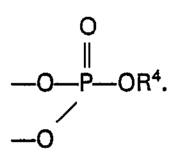

3. Un composé selon la revendication 1 ou 2 ayant pour formule

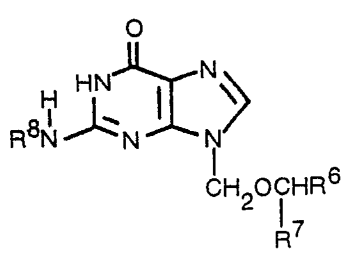

où $R^6$ est $—CH_2OH$ ou

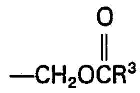 et

$R^7$ est

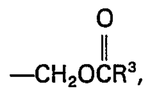 , ou

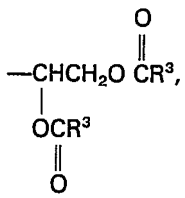 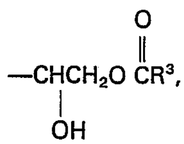 ou 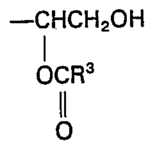

et $R^7$ est H et $R^8$ et $R^3$ sont comme défini dans la revendication 1.

4. Un procédé de préparation d'un composé de la revendication 1 de formule I où $R^1$, $R^2$ et $R^8$ sont chacun H, qui comprend la réaction à une température élevée et sous vide en présence d'un catalyseur acide d'un composé de formule V

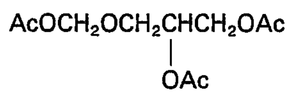 (V)

où Ac est un acétyle, avec un composé de formula VII

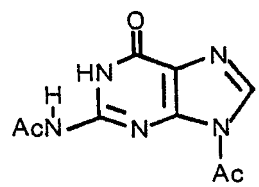 (VII)

où Ac est un acétyle, pour former un composé de formule I est facultativement la désacrylation d'un composé de formule

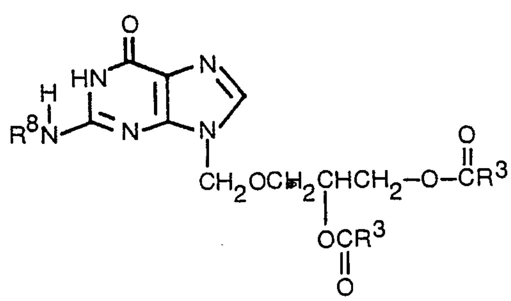

dans laquelle $R^8$ et H ou

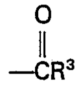

et $R^3$ est comme défini dans la revendication 1.

5. Une procédé de préparation d'un composé selon la revendication 1 de formule II dans laquelle $R^1$, $R^2$ et $R^8$ sont chacun H, qui comprend la réaction à une température élevée et sous vide en présence d'un catalyseur acide d'un composé de formule VI

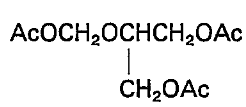 (VI)

où Ac est un acétyle, avec un composé de formule VII come défini dans la revendication 4 pour former un composé de formule I, et facultativement la désacylation d'un composé de formule

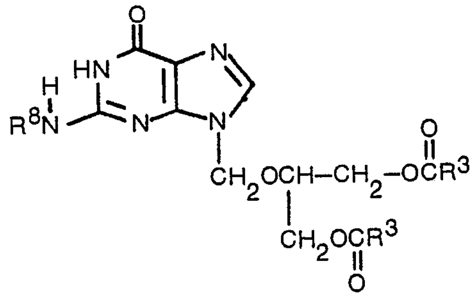

dans laquelle $R^8$ est H ou

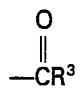

et $R^3$ est comme défini dans la revendication 1.

6. Une procédé de préparation d'un composé de la revendication 3, qui comprend la réaction d'un composé de formule I ou II de la revendication 1, dans laquelle $R^1$, $R^2$ et $R^8$ sont chacun H, avec un composé d'acylation de formule

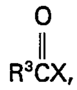

dans laquelle X est un groupe activateur de carboxyle, pour former un dérivé monoacylé lorsqu'on laisse la réaction se poursuivre jusqu'à ce qu'un équivalent du composé d'acylation ait réagi avec le composé de formule I ou II, ou pour former un dérivé diacylé lorsqu'on laisse la réaction se poursuivre jusqu'à ce que deux équivalents du composé d'acylation aient réagi avec le composé de formule I ou II, ou pour former un dérivé triacylé lorsque trois équivlents de l'agent d'acylation aient réagi avec le composé de formule I ou II.

7. Une procédé selon la revendication 6, dans lequel le groupe activateur de carboxyle est un halogénure, un acyloxy, un 1-benzotriazolyloxy ou un N-succinimidyloxy.

8. Un procédé de préparation d'un composé selon la revendication 1, où $R^1$ et $R^2$ sont H ou

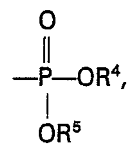

ou $OR^1$ et $OR^2$ ensemble sont

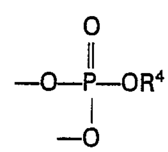

et $R^8$ est H, qui comprend la réaction d'un composé de formule I ou II de la revendication 1, dans laquelle $R^1$, $R^2$ et $R^8$ sont chacun H, avec un agent de phosphorylation et le traitement de l'intermédiaire obtenu avec de l'eau ou un alcanol de 1 à 8 atomes de carbone et l'isolement d'au moins un des dérivés de type phosphate acyclique ou cyclique obtenus.

9. Un procédé selon la revendication 8, dans lequel l'agent de phosphorylation est $POX_3$ où X est un halogène.

10. Un procédé de préparation d'un composé selon la revendication 1, dans lequel un de $R^1$ et $R^2$ est H et l'autre est

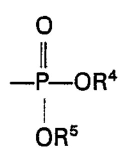

et $R^8$ est H et $R^4$ est comme défini dans la revendication 1, et $R^5$ est soit H, soit un cation convenant en pharmacie qui comprend la réaction d'un composé de formule I ou II dans laquelle $OR^1$ et $OR^2$ sont ensemble

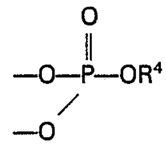

avec un hydroxyde de métal alcalin aqueux.

11. Un procédé de préparation d'un dérivé monophosphate d'un composé de formule I ou II de la revendication 1, où $R^1$, $R^2$ et $R^8$ sont chacun H, qui comprend l'incubation dudit composé de formule I ou II en présence de thymidine kinase d'HSV1 et d'adénosine triphosphate.

12. Un procédé de préparation d'un dérivé pyrophosphate d'un composé de formule I ou II de la revendication 1, où $R^1$, $R^2$ et $R^8$ sont chacun H, qui comprend l'incubation dudit composé de formule I ou II en présence de thymidine kinase d'HSV1, d'adénosine triphosphate et de guanosine-monophosphate kinase.

13. Un procédé de préparation d'un dérivé triphosphate linéaire d'un composé de formule I ou II de la revendication 1, où $R^1$, $R^2$ et $R^8$ sont chacun H, qui comprend l'incubation dudit composé de formule I ou II en présence de thymidine kinase d'HSV1, d'adénosine triphosphate, de guanosine-monophosphate kinase et d'un extrait de cellules infectées par HSV1.

14. Un procédé de préparation d'un dérivé triphosphate linéaire d'un composé de formule I ou II de la revendication 1, où $R^1$, $R^2$ et $R^8$ sont chacun H, qui comprend l'incubation d'un composé de formule I ou II dans laquelle un de $R^1$ ou $R^2$ est

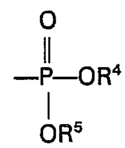

l'autre est H et $R^2$ est un phosphate et $R^8$ est H, avec la 3'-phosphoglycérate kinase, la 3-phosphoglycéraldéhyde déshydrogénase et le 3-phosphoglycéraldéhyde.

15. Un intermédiaire d'un composé de formule I de la revendication 1, de formule

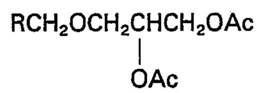

dans laquelle R est AcO ou le brome, le chlore ou l'iode et Ac est un acétyle.

16. Un intermédiaire d'un composé de formule II de la revendication 1, de formule

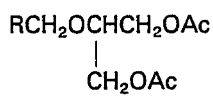

dans laquelle R est AcO ou le brome, le chlore ou l'iode et Ac est un acétyle.

17. Une composition pour traiter une infection par un herpèsvirus chez les mammifères ou les oiseaux, qui contient un composé de formule I ou II

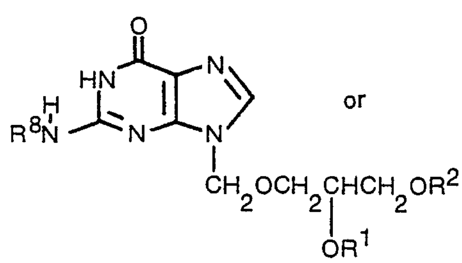

or

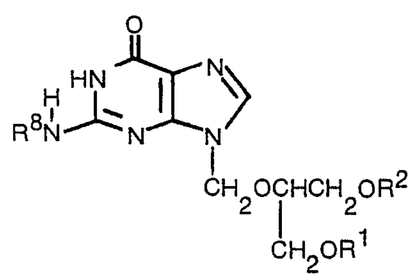

I II

et/ou leurs sels convenant en pharmacie, où $R^1$ et $R^2$ sont indépendamment H,

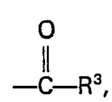

$R^3$ étant comme défini dans la revendication 1,

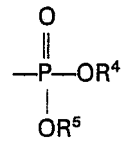

ou $OR^1$ et $OR^2$ ensemble sont

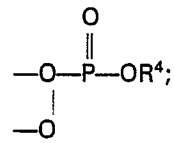

$R^4$ et $R^5$ sont indépendamment H, un cation covenant en pharmacie, un alkyle de 1 à 8 atomes de carbone pouvant être à chaîne droite our ramifiée, un phényle facultativement substitué par un halogènene ou un alkyle en $C_{1-4}$, un phosphate ou un pyrophosphate, et $R^8$ est H ou un acyle, sous réserve que $R^1$, $R^2$ et $R^8$ ne représentent pas simultanément H dans la formule II, en une quantité efficace pour conférer un effet anti-herpèsvirus.

18. Une composition pour le traitement d'une infection à herpèsvirus chez les mammifères ou les oiseaux, qui contient un composé de la revendication 2 en une quantité efficace pour conférer un effet anti-herpèsvirus.

19. Une composition pour le traitement d'une infection à herpèsvirus chez les mammifères ou les oiseaux, qui contient un composé de la revendication 3 en une quantité efficace pour conférer un effet anti-herpèsvirus.

20. Une composition selon l'une des revendications 17 à 19 pour l'administration du composé à raison d'environ 0,01 à environ 200 mg/kg.

21. Une composition selon l'une des revendications 17 à 19 pour l'administration locale du composé à une dose d'environ 0,1% à environ 5% en poids.

22. Une composition selon la revendication 21, dans laquelle la dose est viron 0,25% à environ 3% en poids.

23. Une composition selon l'une quelconque des revendications 17 à 19 pour l'administration orale ou partentérale du composé à une posologie de 0,8 à environ 100 mg/kg.

24. Une composition selon la revendication 23, dans laquelle la posologie est d'environ 5 à environ 50 mg/kg.

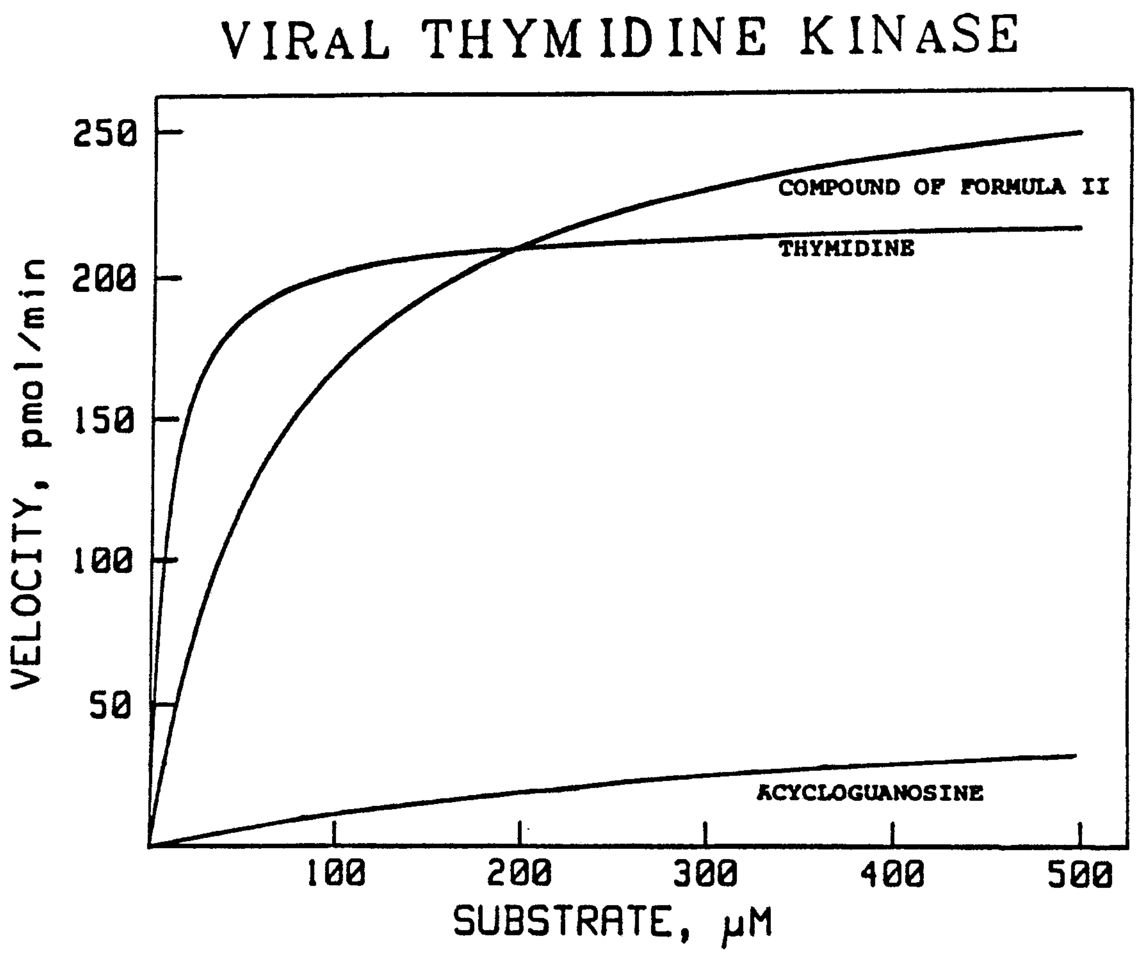
VIRAL THYMIDINE KINASE
VELOCITY, pmol/min
250
200
150
100
50
COMPOUND OF FORMULA II
THYMIDINE
ACYCLOGUANOSINE
100
200
300
400
500
SUBSTRATE, μM

FIG.1

ORAL TREATMENT OF MICE HAVING HSV-1 OROFACIAL INFECTION USING COMPOUND OF FORMULA II OR ACYCLOGUANOSINE
S= SIGNIFICANT PROTECTION ($P < 0.05$)

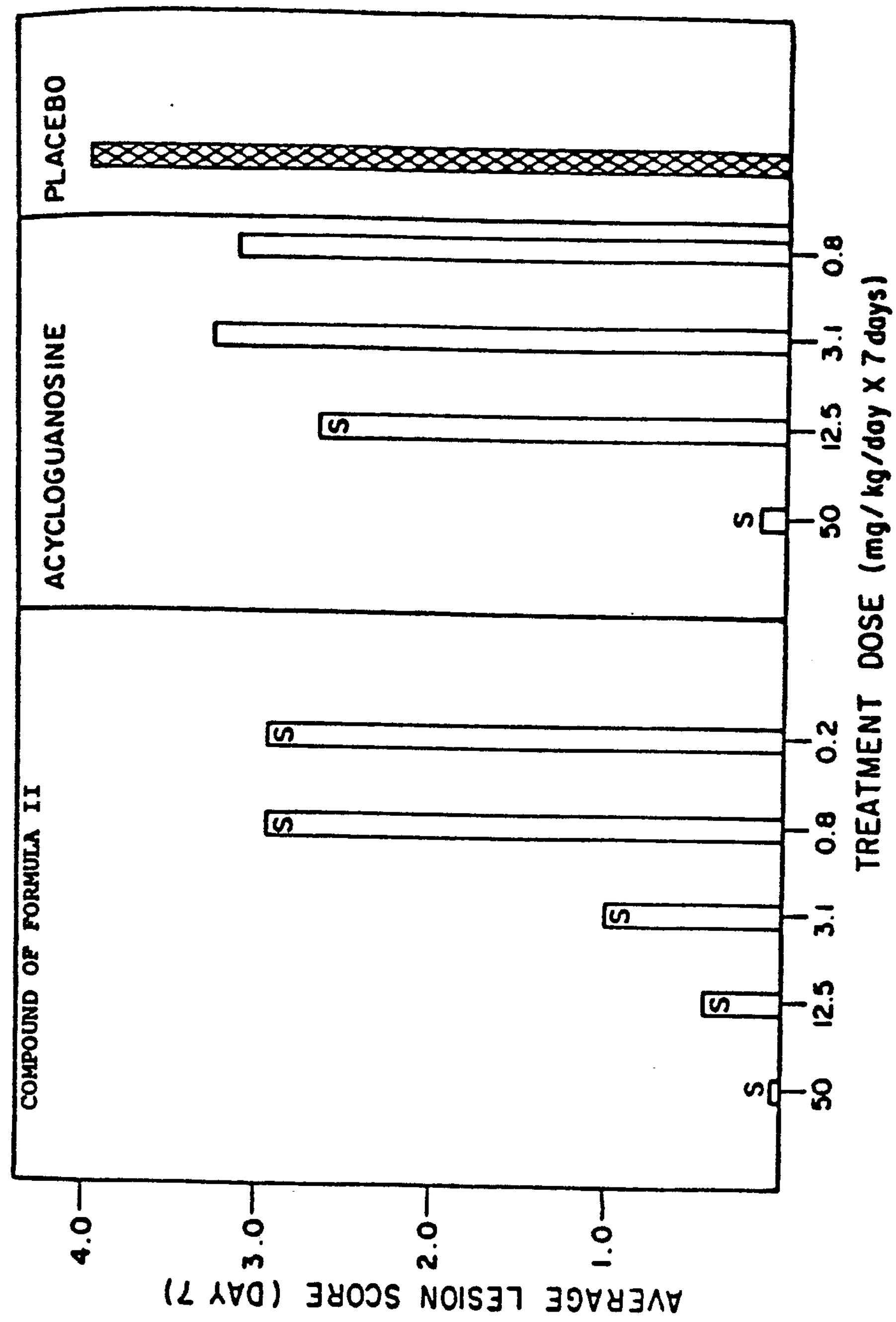


FIG. 2